# EUROPÄISCHE PATENTSCHRIFT

(11) **EP 2 294 159 B1**
(45) Veröffentlichungstag und Bekanntmachung des Hinweises auf die Patenterteilung: **05.09.2012**
(21) Anmeldenummer: 09768868.3
(22) Anmeldetag: 28.04.2009
(51) Int. Cl.: C12Q 1/68, C09K 11/06, G01N 21/64, C07F 5/00, C09B 57/00, G01N 33/542

(54) **ABSTANDSGESTEUERTE ENERGIETRANSFER-FARBSTOFFKOMPLEXE**
DISTANCE-CONTROLLED ENERGY TRANSFER DYE COMPLEXES
COMPLEXES COLORANTS À TRANSFERT D'ÉNERGIE RÉGULÉS PAR L'ÉCART LES SÉPARANT

(30) Priorität: 26.06.2008 DE 102008029936; 18.07.2008 DE 102008033871
(43) Veröffentlichungstag der Anmeldung: 16.03.2011
(73) Patentinhaber: Inbio Prof. Jürgen Büddefeld Dr. Peter Klauth Prof. Manfred Rietz Gbr, 47239 Duisburg (DE)
(72) Erfinder: KLAUTH, Peter, 41189 Mönchengladbach (DE); RIETZ, Manfred, 41812 Erkelenz (DE)
(74) Vertreter: Gesthuysen, von Rohr & Eggert
(86) Internationale Anmeldenummer: PCT/EP2009/003067
(87) Internationale Veröffentlichungsnummer: WO 2009/156019

(56) Entgegenhaltungen:
- WO-A2-2006/116981
- DE-A1- 10 259 677
- JP-A- 56 136 874
- US-A- 5 118 349
- PARK Y J; LEE B H; KIM W H; DO Y: "Investigation of Coordinational Properties of Europium(III) Complexes with Picolinic Acid Using Eu(III) Excitation Spectroscopy" JOURNAL OF COLLOID AND INTERFACE SCIENCE, ACADEMIC PRESS, NEW YORK, NY, US, Bd. 209, Nr. 1, 1. Januar 1999 (1999-01-01), Seiten 268-270, XP026210320 ISSN: 0021-9797 [gefunden am 1999-01-01]
- LI MIN; SELVIN P R: "Amine-reactive forms of a luminescent diethylenetriaminepentaacetic acid chelate of terbium and europium: Attachment to DNA and energy transfer measurements" BIOCONJUGATE CHEMISTRY, ACS, WASHINGTON, DC, US, Bd. 8, Nr. 2, 1. Januar 1997 (1997-01-01), Seiten 127-132, XP002522976 ISSN: 1043-1802
- CHEN JIYAN; SELVIN P R: "Thiol-reactive luminescent chelates of terbium and europium" BIOCONJUGATE CHEMISTRY, ACS, WASHINGTON, DC, US, Bd. 10, Nr. 2, 1. März 1999 (1999-03-01), Seiten 311-315, XP002522978 ISSN: 1043-1802 [gefunden am 1999-05-02]
- LI M; SELVIN P R: "Luminescent polyaminocarboxylate chelates of terbium and europium: the effect of chelate structure" JOURNAL OF THE AMERICAN CHEMICAL SOCIETY, AMERICAN CHEMICAL SOCIETY, WASHINGTON, DC. US, Bd. 117, Nr. 31, 1. Januar 1995 (1995-01-01), Seiten 8132-8138, XP002220630 ISSN: 0002-7863

## Beschreibung

Die vorliegende Erfindung betrifft die Verwendung von mindestens zwei voneinander verschiedenen Fluorophoren zur Ausbildung eines Fluoreszenzresonanzenergietransfer-Paares (FRET-Paares) nach dem Oberbegriff von Anspruch 1.

Zudem betrifft die vorliegende Erfindung einen Fluoreszenzresonanzenergietransfer-Komplex (FRET-Komplex), welcher mindestens einen ersten Fluorophor (A) als Donorfluorophor und mindestens einen zweiten Fluorophor (B) als Akzeptorfluorophor sowie einen speziellen organischen, die Fluorophore (A) und (B) verbindenden Rest aufweist.

Zudem betrifft die vorliegende Erfindung die erfindungsgemäße Verwendung des FRET-Komplexes nach der Erfindung für verschiedene Zwecke, insbesondere zur Detektion eines Ereignisses in einer Probe bzw. zur Interaktion mit einem Zielmolekül bzw. einer Zielstruktur und/oder zur Detektion bzw. Identifikation bzw. zum Nachweis eines Zielmoleküls bzw. einer Zielstruktur.

Des weiteren betrifft die vorliegende Erfindung die Verwendung des FRET-Komplexes nach der Erfindung für weitere Anwendungszwecke, beispielsweise als Farbstoff, insbesondere Fluoreszenzfarbstoff, oder als bzw. in Biosensoren oder als bzw. in (Bio-)Sonden, insbesondere FRET-Sonden.

Metallorganische Komplexe der Seltenen Erden, insbesondere deren β-Diketonate und deren aromatische Carboxylate, haben bereits Anwendungen in verschiedenen Bereichen gefunden, in denen ihr einzigartiger Lumineszenzmechanismus (sogenannter "Antenneneffekt" bzw. Englisch "*antenna effect*") von Vorteil ist, beispielsweise in Biosensoren.

Der Fluoreszenzresonanzenergietransfer (*Fluorescence Resonance Energy Transfer,* FRET) stellt einen im Stand der Technik bekannten physikalischen Prozeß dar, bei dem im allgemeinen Energie eines angeregten Fluoreszenzfarbstoffes, welcher synonym auch als Donor bzw. Donorfluorophor bezeichnet wird, auf einen zweiten Fluoreszenzfarbstoff, welcher synonym auch als Akzeptor bzw. Akzeptor-Fluorophor bzw. Quencher bezeichnet wird, übertragen werden kann. Die Intensität des Energietransfers bzw. des FRET hängt dabei insbesondere vom Abstand der beiden Fluorophore zueinander ab. Das grundlegende Prinzip des FRET ist darin zu sehen, daß zwischen zwei Farbstoffen, die zur Fluoreszenz befähigt sind, die Energie eines angeregten Donor-Fluorophors auf ein Akzeptor-Fluorophor übertragen werden kann, wobei dieser Energieübertrag nicht in Form von Fluoreszenz, sondern strahlungslos, beispielsweise über Dipol/Dipol-Wechselwirkungen, auf den fluoreszierenden Akzeptor erfolgt.

Um eine derartige strahlungsfreie Energieübertragung auf den Akzeptor mittels FRET zu ermöglichen, müssen verschiedene Kriterien erfüllt sein. Zum einen sollte das Emissionsspektrum des Donors mit dem Absorptionsspektrum des Akzeptors überlappen. Zum anderen sollten der Donor einerseits und der Akzeptor andererseits parallele elektronische Schwingungsebenen aufweisen, und schließlich sollten der Donor und der Akzeptor nur einen oder wenige Nanometer voneinander entfernt sein, da die Intensität des FRET-Signals mit der sechsten Potenz des Abstands der beiden Fluorophore zueinander abnimmt. Da die Intensität des FRET unter anderem von der Beabstandung der beiden Fluorophore abhängt, hat sich die Nutzung von FRET gewissermaßen als optisches Nanometermaß insbesondere in der Biochemie und der Zellbiologie etabliert, wobei in diesem Zusammenhang insbesondere ein Nachweis von Protein/Protein-, Protein/Nukleinsäure- und Nukleinsäure/Nukleinsäure-Wechselwirkungen sowie räumlichen Strukturänderungen, wie Konformationsänderungen von Biomolekülen, detektiert werden können. In diesem Zusammenhang kann auf Basis des FRET-Phänomens auch ein Nachweis einer Interaktion, Homo- und Heterodimerisierung bzw. Oligomerisierung von Proteinen erfolgen. Auch die Vermessung von Konformationsänderungen von Proteinen ist möglich, wenn Donor- und Akzeptorfluorophor an dasselbe Molekül gekoppelt sind. Gleichermaßen wird das Prinzip des FRET in der Molekularbiologie bei der Quantifizierung von Nukleinsäuren mit Hilfe der Real-time-PCR (Realtime-Polymerase Chain Reaction) über den Einsatz spezieller Sonden in der Praxis eingesetzt.

In diesem Zusammenhang besteht eine Möglichkeit der Nutzung des FRET zur Quantifizierung von Nukleinsäuren in der Verwendung von sogenannten LightCycler^{®}-Sonden, welche spezielle Hydrolysesonden darstellen, wobei verschiedene, jeweils mit einem Donor bzw. Akzeptor markierte Oligonukleotide, die nebeneinander an die Zielsequenz eines Nukleinsäuremoleküls binden und damit den Donor und den Akzeptor in eine für den FRET ausreichende Nähe bringen, als Sonden für die Quantifizierung von PCR-Produkten eingesetzt werden.

Eine weitere häufig genutzte Möglichkeit des FRET besteht in der Anwendung sogenannter TaqMan^{®}-Sonden, welche spezielle Hydrolyse-Sonden darstellen, wobei die Sonde an ihrem einen Ende mit einem Akzeptor bzw. Quencher und an ihrem anderen Ende mit einem Donor- bzw. Reporterfluoreszenzfarbstoff markiert ist. Die Fluoreszenz des Donorfluorophors wird bei intakten Hydrolyse-Sonden durch den Quencher durch strahlungsfreie Energieübertragung (FRET) unterdrückt. Während eines PCR-Zyklus hybridisiert die Sonde mit dem komplementären DNA-Strang, die Donorfluoreszenz bleibt zunächst unterdrückt. Die Taq-Polymerase baut das 5'-Ende der Sonde während der PCR-Zyklen ab. Die Fluoreszenz des Donors wird nun nicht mehr durch den Quencher gelöscht und kann gemessen werden.

Eine weitere Möglichkeit der Echtzeit-Quantifizierung von PCR-Produkten unter Ausnutzung des FRET bietet die Nutzung von sogenannten Molecular Beacons als Sonden. Molecular Beacons sind Oligonukleotide, die sowohl mit einem Donor als auch mit einem Quencher bzw. Akzeptor gekoppelt sind. Die Nukleotide am 5'-Ende der Sonde sind zu den Nukleotiden am 3'-Ende komplementär, so daß sich eine für Molecular Beacons charakteristische, schleifenartige Sekundärstruktur ausbilden kann. In diesem als Stem Loop (Stamm-Schleife) bezeichneten Zustand zeigt der Donor durch seinen geringen Abstand zum Akzeptor keine Fluoreszenz. Durch Anlagerung der Schleifenregion an eine komplementäre DNA-Sequenz während eines PCR-Zyklus wird der Abstand zwischen Donor und Akzeptor vergrößert, so daß eine Donorfluoreszenz beobachtet werden kann.

Die Struktur und das Funktionsprinzip der Molecular Beacons sind ausführlich beschrieben von Tyagi und Kramen (1996): "Molecular beacons: probes that fluoresce upon hybridization" in Nature Biotechnology 14, 303-308. Ebenso sind sie Gegenstand der US-Patentschrift 5,925,517, die hiermit zu Offenbarungszwecken hinsichtlich der Struktur und Funktion von Molecular Beacons vollumfänglich in Bezug genommen wird.

Als Fluorophor/Quencher-Paar ist die 5'-(2-Aminoethyl)-aminonaphthalin-1-sulfonsäure (EDANS) am 5'-Ende und als Quencher die 4-(4'-Dimethylamino)phenylazobenzoesäure (DABCYL) am 3'-Ende bekannt. Ebenso ist auch die Verwendung von DABCYL in Kombination mit Fluorescein am 5'-Ende geeignet (G. Leone, von Schijndel H., van Gemen B., Kramen R. F. und Schön D. (1998) "Molecular Beacon Probes Combined with Amplification by NASBA Enable Homogeneous Real-time Detection of RNA" in: Nucleic Acids Research 26, 9, 2150-2155).

Da Molecular Beacons aufgrund des durch FRET verursachten Quenching im Ergebnis keine oder kaum eine Fluoreszenzstrahlung emittieren oder eine spektrale Verschiebung bei Verwendung von zwei Fluorophoren verursachen und damit das Signal aus dem Meßbereich entfernen, wenn die Probensequenz nicht mit der Zielsequenz hybridisiert vorliegt, haben sich Molecular Beacons in der jüngeren Vergangenheit besonders als Sonden in Nachweissystemen bewährt, in denen das Auswaschen überschüssiger Sonden nicht gewünscht oder möglich ist. Dies gilt insbesondere bei der Realtime-PCR oder bei der Detektion von Nukleinsäuren in lebenden Zellen (Liu X., Farmerie W., Schuster S., Tan W. (2000): "Molecular beacons for DNA Biosensors with a Micrometer to Submicrometer Dimension" in: Analytical Biochemistry 283, 56-63).

Gerade in komplexen biologischen Systemen, wie lebenden Zellen, hat sich jedoch die unspezifische Fluoreszenz beispielsweise zellulärer Bestandteile als wesentliche systematische Fehlerquelle erwiesen, welche die Aussagekraft und Spezifität der Nachweismethoden selbst mit den bekannten Molecular Beacons bzw. FRET-Sonden im allgemeinen deutlich vermindert. So zeigen beispielsweise Hämoglobine oder aromatische Kohlenwasserstoffe bei Anregung mit UV-Licht eine unspezifische Fluoreszenz, die sich als Hintergrundstrahlung negativ bemerkbar macht. Das eigentliche Meßsignal wird somit überlagert. Diese Hintergrundstrahlung wird auch als Autofluoreszenz der Probenumgebung oder des Probenmilieus bezeichnet.

Im Rahmen des FRET-Prozesses, insbesondere im Hinblick auf seine molekularbiologische Anwendung, besteht somit ein großer Bedarf, die Leistungsfähigkeit für die jeweiligen FRET-Sonden zu erhöhen. Dabei kommt nicht nur der konkreten Auswahl und Abstimmung der eingesetzten Farbstoffe eine große Bedeutung zu, sondern auch der Ausbildung der Sonde vor dem Hintergrund der räumlichen Beabstandung der Farbstoffe zueinander. Denn, wie zuvor angeführt, das FRET-Phänomen findet nur bei sehr kleinen Beabstandungen zwischen Donor und Akzeptor statt und nimmt mit der sechsten Potenz des Abstandes der Farbstoffe bzw. Fluorophore ab. Insbesondere vor diesem Hintergrund ist es bislang im Stand der Technik nicht immer im vollen Umfang gelungen, leistungsfähige Sonden, insbesondere Molecular Beacons, bereitzustellen. Denn die Sonden des Standes der Technik weisen nicht immer ein optimales Emissionsspektrum auf, insbesondere was die Differenzierung des Emissionsspektrums gegenüber der Autofluoreszenz einer Meßprobe bzw. eines Probenmilieus anbelangt. Zudem sind die Emissionszeiten oftmals nicht optimal insofern, als diese in der Regel bereits nach einigen Nanosekunden nach erfolgter Anregung abklingen, so daß im Rahmen einer zeitaufgelösten Messung die Erfassung der Emission der FRET-Sonde zeitgleich zu dem Anregungssignal erfolgen muß, was hinsichtlich der Erfassung und Auswertung des Signals ungünstig ist. Zudem liegt bei den im Stand der Technik bekannten FRET-Sonden nicht immer ein optimaler FRET vor, so daß das Quenchen des Donors nicht immer optimal ist.

Das auf den Anmelder selbst zurückgehende deutsche Patent DE 102 59 677 B4 betrifft ein Verfahren zur Identifizierung und Quantifizierung von Mikroorganismen in einer Probe, wobei nach Isolierung der Mikroorganismen und Überführung der isolierten Mikroorganismen in ein PCR-Gefäß eine Amplifikation der Zielnukleinsäure erfolgt und ein Nachweis der Zielnukleinsäure mittels einer speziellen Sonde mit mindestens zwei ein FRET-Paar bildenden Sonden erfolgt. Die eingesetzte FRET-Sonde emittiert erst nach der Hybridisierung der Sonde mit dem Zielnukleotid ein erkennbares Signal, welches eine gegenüber der Fluoreszenzemission der Probenumgebung verlängerte Lebensdauer aufweist, so daß das Meßsignal durch eine zeitaufgelöste Fluoreszenzmessung von der Autofluoreszenz der Probenumgebung differenzierbar ist. In diesem Zusammenhang wird hinsichtlich der eingesetzten FRET-Sonde vorrangig auf ein spezielles FRET-Paar auf Basis von Euttaphen und Cy5 abgestellt. Eine derartige Kombination von Fluoreszenz-Farbstoffen weist zwar gegenüber dem Stand der Technik eine deutliche Verbesserung hinsichtlich des Emissionsverhaltes auf, jedoch sind die diesbezüglichen Eigenschaften nicht immer optimal. Insbesondere handelt es sich bei den in dem deutschen Patent beschriebenen Sonden um ein sogenanntes Einfarben-Assay, welches nur im begrenzten Umfang eine Aussage, insbesondere hinsichtlich der Beabstandung der entsprechenden Fluoreszenzfarbstoffe zueinander, ermöglicht.

Die Druckschrift US 5 118 349 A betrifft Sicherheitsfasern und Textilfasern, welche mindestens ein Markierungsprodukt aufweisen. Das Markierungsprodukt enthält Chelate der Seltenen Erden oder Substrate auf Basis von Chelaten der Seltenen Erden, wobei das in den Chelaten vorhandene Ion der Seltenen Erden aus zwei voneinander verschiedenen Seltenen Erden ausgewählt ist. Ein zwischen beiden Seltenen Erden stattfindender Energietransfer ruft eine Wellenlängenänderung der Fluoreszenz der Chelate hervor, sofern diese ultravioletter Strahlung ausgesetzt werden, wobei die Änderung von der Temperatur des Chelats abhängt. Das Markierungsprodukt wird in Fasern bzw. Garne mittels eines Färbeverfahrens eingefügt, wobei die Fasern bzw. Garne bei Bestrahlung mit Sonnen- oder Kunstlicht farblos sind und unter UV-Bestrahlung ihre Fluoreszenzwellenlänge in Abhängigkeit von der Temperatur zu ändern imstande sind.

Vor diesem Hintergrund besteht die Aufgabe der vorliegenden Erfindung darin, einen FRET-Komplex bereitzustellen, welcher sich für die vorgenannten Anwendungen eignet und insbesondere die Nachteile des Standes der Technik zumindest teilweise vermeidet oder aber wenigstens abschwächt.

Insbesondere besteht eine Aufgabe der vorliegenden Erfindung darin, einen FRET-Komplex bereitzustellen, welcher sich beispielsweise für molekularbiologische Analyseverfahren zum qualitativen und quantitativen Nachweis von Zielmolekülen, wie Nukleinsäuremolekülen, beispielsweise im Rahmen einer Realtime-PCR, sowie für den Nachweis von Strukturänderungen beispielsweise bei Proteinen, eignet. Dabei soll insbesondere ein FRET-Komplex mit einem verbesserten Emissionsverhalten, insbesondere hinsichtlich Emissionsdauer und Breite der Emissionsbanden, bereitgestellt werden, wobei insbesondere eine bessere Differenzierbarkeit bzw. Diskriminierung gegenüber der Autofluoreszenz einer Probe ermöglicht werden soll. Zudem sollen auf Basis des erfindungsgemäßen FRET-Komplexes verbesserte qualitative Aussagen hinsichtlich der Beabstandung der Farbstoffkomplexe und somit hinsichtlich der Wechselwirkung mit dem Zielmolekül bzw. der Zielstruktur ermöglicht werden.

Zur Lösung der zuvor geschilderten Aufgabenstellung schlägt die vorliegende Erfindung - gemäß einem ersten Aspekt der vorliegenden Erfindung - die Verwendung von mindestens zwei voneinander verschiedenen Fluorophoren zur Ausbildung eines Fluoreszenzresonanzenergietransfer-Paares (FRET-Paares) gemäß Anspruch 1 vor. Weitere vorteilhafte Ausgestaltungen der erfindungsgemäßen Verwendung sind Gegenstand der diesbezüglichen Unteransprüche.

Weiterer Gegenstand der vorliegenden Erfindung - gemäß einem zweiten Aspekt der vorliegenden Erfindung - ist der erfindungsgemäße Fluoreszenzresonanzenergietransfer-Komplex (FRET-Komplex) gemäß Anspruch 9.

Weiterer Gegenstand der vorliegenden Erfindung - gemäß einem dritten Aspekt der vorliegenden Erfindung - ist die erfindungsgemäße Verwendung des FRET-Komplexes nach der Erfindung zur Detektion eines Ereignisses in einer Probe gemäß Anspruch 10.

Wiederum weiterer Gegenstand der vorliegenden Erfindung - gemäß einem vierten Aspekt der vorliegenden Erfindung - ist die erfindungsgemäße Verwendung des FRET-Komplexes nach der Erfindung zur Interaktion mit einem Zielmolekül bzw. einer Zielstruktur gemäß Anspruch 11.

Des weiteren ist ein weiterer Gegenstand der vorliegenden Erfindung - gemäß einem fünften Aspekt der vorliegenden Erfindung - die erfindungsgemäße Verwendung des FRET-Komplexes nach der Erfindung als Farbstoff für Markierungszwecke oder dergleichen gemäß Anspruch 12.

Weiterhin ist ein weiterer Gegenstand der vorliegenden Erfindung - gemäß einem sechsten Aspekt der vorliegenden Erfindung - die erfindungsgemäße Verwendung des FRET-Komplexes nach der Erfindung in oder als Biosensoren oder aber in oder als (Bio-)Sonden, insbesondere FRET-Sonden, gemäß Anspruch 13.

Es versteht sich von selbst, daß Ausgestaltungen, Ausführungsformen, Vorteile und dergleichen, welche nachfolgend zu Zwecken der Vermeidung von Wiederholungen nur zu einem Erfindungsaspekt aufgeführt sind, selbstverständlich auch in bezug auf die übrigen Erfindungsaspekte entsprechend gelten.

Der Anmelder hat nun in überraschender Weise herausgefunden, daß das zuvor geschilderte Problem dadurch gelöst werden kann, daß man mindestens zwei spezielle, voneinander verschiedene Fluorophore, wie nachfolgend definiert, zur Ausbildung eines Fluoreszenzresonanzenergietransfer-Paares (synonym auch als FRET-Paar bezeichnet) verwendet. Das FRET-Paar weist dabei einen ersten Fluorophor (A) als Donorfluorophor und mindestens einen zweiten Fluorophor (B) als Akzeptorfluorophor auf. Die erfindungsgemäße Verwendung zeichnet sich dadurch aus, daß der erste Fluorophor (A) und der zweite Fluorophor (B), unabhängig voneinander, jeweils auf Basis eines Organometallkomplexes von Elementen der Seltenen Erden ausgebildet sind. Dabei werden die Fluorophore (A) und (B) im Rahmen der vorliegenden Erfindung derart ausgewählt, daß sie voneinander verschiedene Elemente der Seltenen Erden aufweisen.

Das im Rahmen der erfindungsgemäßen Verwendung ausgebildete FRET-Paar eignet sich insbesondere zur Verwendung im Rahmen von biochemischen bzw. molekularbiologischen Analyseverfahren, z. B. zur Detektion von spezifischen Zielmolekülen bzw. Zielstrukturen, insbesondere Biomolekülen, wie DNA, RNA, Proteinen und dergleichen, in einer Probe und somit gewissermaßen als FRET-Sonde, wobei in diesem Fall das im Rahmen der erfindungsgemäßen Verwendung ausgebildete FRET-Paar zudem spezifische Abschnitte bzw. Bestandteile aufweisen kann, welche zur Wechselwirkung mit dem Zielmolekül bzw. der Zielstruktur imstande sind. Zudem eignet sich das im Rahmen der vorliegenden Erfindung bereitgestellte FRET-Paar zur Erfassung bzw. Detektion von Konformationsänderungen bei Molekülen, beispielsweise von Proteinen oder Nukleinsäuren. Auch Spaltungs- und Anlagerungsvorgänge von Biomolekülen können mit dem erfindungsgemäß bereitgestellten FRET-Paar untersucht werden. Zudem ist auch ein Einsatz als Farbstoff möglich.

Im Rahmen der vorliegenden Erfindung ist es dabei gelungen, durch die spezifische Auswahl der Fluorophore (A) und (B) mit jeweils voneinander verschiedenen Elementen der Seltenen Erden sowie der weiteren speziellen und im nachfolgenden noch beschriebenen Ausgestaltung des FRET-Systems, insbesondere hinsichtlich der räumlichen Anordnung der Fluorophore (A) und (B) zueinander, ein leistungsfähiges System bzw. einen Komplex bereitzustellen, welcher sich im Rahmen einer FRET-Analyse bzw. FRET-Untersuchung in hohem Maße zum Einsatz insbesondere in der molekularbiologischen bzw. biochemischen Analyse zur Detektion bzw. Auffindung sowie zur Analyse von räumlichen Änderungen von spezifischen Zielmolekülen bzw. Zielstrukturen eignet.

Dabei zeichnen sich die im Rahmen der vorliegenden Erfindung bereitgestellten FRET-Paare dadurch aus, daß sie ein definiertes Emissionsspektrum mit einer sehr schmalen Emissionsbande aufweisen, was zu einer hervorragenden Detektierbarkeit führt, insbesondere da sich das Emissionsspektrum der erfindungsgemäß bereitgestellten FRET-Paare signifikant von der unspezifischen Fluoreszenzemission einer Probenumgebung und somit von der Autofluoreszenz unterscheidet, so daß eine hervorragende Diskriminierung des gewünschten Meßsignals gegenüber der unspezifischen Hintergrundstrahlung vorliegt.

Diese Eigenschaft der erfindungsgemäß bereitgestellten FRET-Paare bzw. FRET-Sonden und somit gleichermaßen der nachfolgend noch beschriebenen FRET-Komplexe wird dadurch noch weiter verbessert, daß die erfindungsgemäß bereitgestellten FRET-Paare eine sehr lange Emissionsdauer aufweisen, d. h die erfindungsgemäß bereitgestellten FRET-Paare bzw. FRET-Sonden geben nach erfolgter Anregung durch ein entsprechend geeignetes Anregungssignal ein zeitlich verlängertes Emissionssignal ab, was die Detektierbarkeit weiter verbessert. So beträgt die Zeitdauer der Signalemission des erfindungsgemäß bereitgestellten FRET-Paares mehrere Millisekunden, während beispielsweise die unerwünschte Störfluoreszenz auf Basis einer Autofluoreszenz der Probenumgebung bereits im Bereich von Nanosekunden (beispielsweise etwa 200 Nanosekunden) nach Beendigung eines Anregungssignals abklingt. Hieraus resultiert eine weitere Verbesserung der Differenzierbarkeit bzw. Diskriminierung des Meßsignals einerseits zum Störsignal andererseits.

Ein weiterer entscheidender Vorteil des erfindungsgemäß bereitgestellten FRET-Paares ist darin zu sehen, daß es sich hierbei aufgrund der spezifischen Auswahl der Fluorophore (A) und (B), welche voneinander verschiedene Elemente der Seltenen Erden aufweisen, um sogenannte Zweifarben-Assays handelt, welche zur qualitativen und quantitativen Analyse von Wechselwirkungen bzw. Strukturänderungen und dergleichen eingesetzt werden können. So kann das erfindungsgemäß bereitgestellte FRET-System unter Verwendung spezieller Terbium- und Europium-Komplexe für die Fluorophore (A) und (B) bei geringer Beabstandung der Fluorophore (A) und (B) zueinander und somit bei maximalem Energietransfer rot lumineszieren, während bei großer Beabstandung der Fluorophore eine gelbe Lumineszenz vorliegt. In den Zwischenzuständen - also bei mittleren Abständen der Fluorophore - luminesziert das erfindungsgemäß bereitgestellte FRET-Paar bzw. die erfindungsgemäß bereitgestellte FRET-Sonde im orangefarbenen Farbbereich, und zwar mit einem entsprechenden farblichen Übergang von rot nach gelb, je nach Beabstandung der Fluorophore zueinander. Dies ist insbesondere auch für den Einsatz der erfindungsgemäß bereitgestellten FRET-Paare als Farbstoffe von Vorteil.

Vor diesem Hintergrund eignet sich das im Rahmen der vorliegenden Erfindung bereitgestellte FRET-Paar bzw. die FRET-Sonde und somit das FRET-System bzw. der erfindungsgemäße FRET-Komplex insbesondere zum Einsatz im Bereich der Biochemie bzw. Molekularbiologie und der Zellbiologie. Diesbezüglich eignet sich das erfindungsgemäß bereitgestellte FRET-Paar insbesondere zum Nachweis von Protein/Protein-, Protein/Nukleinsäure- und Nukleinsäure/Nukleinsäure-Wechselwirkungen sowie Konformationsänderungen und Spaltungsvorgängen von bzw. bei Biomolekülen, wobei aufgrund der spezifischen Ausbildung als Zweifarb-Assay sogar eine quantitative Analyse der zugrundeliegenden Ereignisse erfolgen kann. Insbesondere eignet sich das erfindungsgemäß bereitgestellte FRET-System im Rahmen der Quantifizierung von Nukleinsäuren, beispielsweise im Rahmen einer Realtime-PCR.

Gegenstand der vorliegenden Erfindung - gemäß einem ersten Aspekt der vorliegenden Erfindung - ist somit die Verwendung von mindestens zwei voneinander verschiedenen Fluorophore zur Ausbildung eines Fluoreszenzresonanzenergietransfer-Paares (FRET-Paares), wobei innerhalb des FRET-Paares mindestens ein erster Fluorophor (A) als Donor-Fluorophor und mindestens ein zweiter Fluorophor (B) als Akzeptor-Fluorophor dient. Die erfindungsgemäße Verwendung zeichnet sich dadurch aus, daß der erste Fluorophor (A) und der zweite Fluorophor (B), unabhängig voneinander, jeweils auf Basis eines Organometallkomplexes von Elementen der Seltenen Erden ausgebildet sind, wobei die Fluorophore (A) und (B) voneinander verschiedene Elemente der Seltenen Erden, ausgewählt aus der Gruppe von Europium und Terbium, aufweisen. Weiterhin sind die Elemente der Seltenen Erden in den Flurophoren (A) und (B) jeweils mit mindestens einem Liganden in Form eines Komplex- und/oder Chelatbildners komplexiert, wobei der Ligand Picolinsäure, Picolinate und/oder deren Derivate ist und wobei der Ligand Substituenten und/oder funktionelle Gruppen zur vorzugsweise kovalenten Bindung und/oder Kopplung von vorzugsweise organischen Molekülen aufweist, wobei die Substituenten und/oder funktionellen Gruppen ausgewählt sind aus der Gruppe von Amino-, Carboxylat-, Isocyanat-, Thioisocyanat-, Epoxy-, Thiol-und Hydroxygruppen. Zudem sind der Fluorophor (A) und der Fluorophor (B) über einen zweiwertigen und/oder zweibindigen organischen Rest miteinander gekoppelt und/oder verbunden.

Das grundlegende Prinzip der vorliegenden Erfindung ist somit darin zu sehen, daß in bezug auf das FRET-Paar ein erster Fluorophor (A) als Donor eingesetzt wird, welcher ein Zentralatom aus der Gruppe der Seltenen Erde aufweist. Zudem wird erfindungsgemäß ein zweiter Fluorophor (B) eingesetzt, welcher gleichermaßen als Zentralatom ein Element der Seltenen Erden umfaßt, wobei es sich hierbei um ein in bezug auf den ersten Fluorophor (A) unterschiedliches Element handelt. Der gezielte Einsatz von Elementen der Seltenen Erden in bezug auf das erste Fluorophor (A) und das zweite Fluorophor (B) mit der Maßgabe, in den jeweiligen Fluorophoren unterschiedliche Elemente aus dieser Gruppe einzusetzen, führt in völlig überraschender Weise zu den hervorragenden Eigenschaften des im Rahmen der erfindungsgemäßen Verwendung bereitgestellten FRET-Paares hinsichtlich seiner Emissionseigenschaften, wie enge Emissionsbanden, lange Emissions- bzw. Lumineszenzdauern, quantitative Änderung des Emissionsspektrums in Abhängigkeit von der Entfernung der Fluorophore (A) und (B) zueinander im Rahmen eines Zweifarben-Assay und hervorragender FRET bei geringer Beabstandung der Fluorophore zueinander mit optimalen Quenchern des Donorsignals.

Im Rahmen der vorliegenden Erfindung hat es sich als besonders vorteilhaft herausgestellt, wenn der erste Fluorophor (A) und der zweite Fluorophor (B) ausgewählt sind derart, daß unter Einfluß und/oder Einwirkung und/oder Eintrag einer Anregungsenergie ein Energietransfer von dem ersten Fluorophor (A) auf den zweiten Fluorophor (B) erfolgt, insbesondere wobei der Energietransfer von dem ersten Fluorophor (A) auf den zweiten Fluorophor (B) zumindest im wesentlichen strahlungsfrei erfolgt. Ein derartiger Energietransfer liegt insbesondere bei einer geringen Beabstandung der Fluorophore zueinander im Sinne eines FRET vor. Mit zunehmender Beabstandung nimmt der FRET ab. Was die Anregungsenergie anbelangt, so kann diesbezüglich insbesondere eine elektromagnetische Strahlung mit auf den Donor optimierter bzw. ausgerichteter Wellenlänge eingesetzt werden. Dies ist dem Fachmann an sich bekannt.

Dabei kann der Energietransfer von dem ersten Fluorophor (A) auf den zweiten Fluorophor (B) beispielsweise mittels Dipol/Dipol-Wechselwirkungen erfolgen. Das Vorliegen eines Energietransfers kann insbesondere über eine Abnahme der Fluoreszenz des Fluorophors (A) bzw. des Donor-Fluorophors und/oder über eine Zunahme der Fluoreszenz des zweiten Fluorophors (B) bzw. des Akzeptor-Fluorophors detektiert werden.

Als Detektions- bzw. Meßeinheiten kommen in nichtbeschränkender Weise Fluoreszenzmikroskope, Fluorimeter oder dergleichen in Betracht.

Was den Energietransfer von dem ersten Fluorophor (A) auf den zweiten Fluorophor (B) anbelangt, so kann dieser von der Beabstandung der Fluorophore (A) und (B) zueinander abhängig sein. In diesem Zusammenhang kann der Energietransfer, insbesondere die Intensität und/oder Effizienz des Energietransfers, mit abnehmender räumlicher Beabstandung der Fluorophore (A) und (B) zueinander zunehmen. Mit anderen Worten führt somit eine Vergrößerung der Beabstandung des ersten Fluorophors (A) zu dem zweiten Fluorophor (B) zu einer Verringerung des Energietransfers, einhergehend mit einer Änderung des Emissionsspektrums. Eine derartige Änderung der Beabstandung der Fluorophore (A) und (B) zueinander kann beispielsweise im Rahmen einer Wechselwirkung bzw. Interaktion bzw. Anlagerung des FRET-Paares an ein Zielmolekül bzw. an eine Zielstruktur erfolgen, wobei diese Wechselwirkung bzw. Interaktion bzw. Anlagerung an die Zielsstruktur die Abstandsveränderung, insbesondere die Abstandsvergrößerung, der beiden Fluorophore (A) und (B) zueinander, hervorruft, insbesondere vergrößert. Das zugrundeliegende Ereignis ist dann in einer Änderung des Emissionsspektrums erkennbar, wobei mit zunehmender Beabstandung das Donorsignal zunimmt und/oder das Akzeptorsignal abnimmt. Bei Verringerung des Abstandes nimmt entsprechend durch den Quencheffekt des Akzeptors das Donorsignal ab und das Akzeptorsignal zu.

In diesem Zusammenhang kann somit der Energietransfer von dem ersten Fluorophor (A) auf den zweiten Fluorophor (B) zu einer Abnahme der Fluoreszenz des ersten Fluorophors (A) und zu einer Zunahme der Fluoreszenz des zweiten Fluorophors (B) führten.

Erfindungsgemäß ist es vorteilhaft, wenn der Fluorophor (A) und der Fluorophor (B) zumindest im wesentlichen parallele elektronische Schwingungsebenen aufweisen, was insbesondere dann von Bedeutung ist, wenn der Fluorophor (A) einerseits und der Fluorophor (B) andererseits fixiert sind (z. B. an demselben Protein). Auf diese Weise kann bei Veränderung der Position der Fluorophore (A) und (B) zueinander im Rahmen einer Interaktion mit einem Protein bzw. einer Konformationsänderung des Proteins selbst eine Änderung des FRET-Signals Aufschluß über die Änderung der Schwingungsebenen und damit der Lage beider Fluorophore zueinander geben.

Wie zuvor angeführt, sollten zur Ermöglichung eines Energietransfers, insbesondere eines Fluoreszenzresonanzenergietransfers, der Fluorophor (A) und der Fluorophor (B) nur gering voneinander beabstandet sein, wobei die Beabstandung im allgemeinen wenige Nanometer betragen sollte. Denn die Intensität des FRET-Signals nimmt mit der sechsten Potenz des Abstands der Fluorophore zueinander ab. In diesem Zusammenhang ist die Effizienz des Energietransfers vom sogenannten Transfer-Radius des Fluorophoren-Paars abhängig. Das Kriterium des geringen Abstandes zwischen dem Fluorophor (A) und dem Fluorophor (B) führt dazu, daß das im Rahmen der erfindungsgemäßen Verwendung bereitgestellte FRET-Paar gewissermaßen als "optisches Nanometermaß" fungieren kann und sich somit im Rahmen molekularbiologischer bzw. biochemischer Methoden beispielsweise zur qualitativen und quantitativen Bestimmung der Beabstandung der Fluorophore eignet, um auf diese Weise qualitative und quantitative Rückschlüsse über zugrundeliegende Primärereignisse, wie z. B. Interaktion mit einem Zielmolekül bzw. einer Zielstruktur sowie Konformationsänderungen, ziehen zu können.

Hinsichtlich des Energietransfers, insbesondere Fluoreszenzresonanzenergietransfers, insbesondere bei geringer Beabstandung der Fluorophore (A) und (B), kann es auch vorgesehen sein, daß die Zustände des ersten Fluorophors (A) mit den Zuständen des zweiten Fluorophors (B) zumindest teilweise überlappen.

Des weiteren sollte der erste Fluorophor (A) und/oder der zweite Fluorophor (B), unabhängig voneinander, jeweils einen Farbstoff, insbesondere einen Fluoreszenzfarbstoff, darstellen und/oder umfassen.

Im Rahmen der vorliegenden Erfindung ist es vorteilhaft, wenn der erste Fluorophor (A) und/oder der zweite Fluorophor (B), unabhängig voneinander, jeweils mindestens einen Kern, vorzugsweise einen Kern, aus einem Element der Seltenen Erden aufweisen. Dabei ist es besonders bevorzugt, wenn der erste Fluorophor (A) und/oder der zweite Fluorophor (B), unabhängig voneinander, in Form eines Koordinationskomplexes bzw. einer Koordinationsverbindung oder in Form einer Chelatverbindung vorliegen, wobei das bzw. die Zentralatom(e) durch das Element der Seltenen Erden gebildet wird bzw. werden. Dabei ist es im Rahmen der vorliegenden Erfindung besonders bevorzugt, wenn - sofern mehrkernige Koordinationskomplexe eingesetzt werden - innerhalb eines Komplexes und somit innerhalb eines Fluorophors Kerne auf Basis desselben Elementes der Seltenen Erden verwendet werden.

Im Rahmen der vorliegenden Erfindung ist es vorgesehen, daß das Element der Seltenen Erden ausgewählt ist aus den Elementen der Lanthanoide, nämlich aus der Gruppe von Europium und Terbium.

Die Lanthanoide sind silbrig glänzende, relativ weiche und reaktionsfreudige Metalle, die an der Luft schnell oxidieren und dabei matt werden. In Wasser zersetzen sie sich mehr oder weniger schnell unter Freisetzung von Wasserstoffgas. Bei den Lanthanoiden handelt es sich im allgemeinen um insgesamt 14 Elemente der sechsten Periode des Periodensystems, die als Untergruppe der dritten Nebengruppe aufgefaßt werden können. Aufgrund der ähnlichen Struktur der Valenzschale verhalten sich die Lanthanoide chemisch vergleichbar zu den Elementen der dritten Gruppe des Periodensystems, nämlich Skandium und Yttrium. Mit diesen bilden die Lanthanoide die Gruppe der Seltenen Erden.

Besonders gute Ergebnisse hinsichtlich des im Rahmen der vorliegenden Erfindung bereitgestellten FRET-Paares werden erhalten, wenn das Element der Seltenen Erden ausgewählt ist aus der Gruppe von Europium und Terbium.

Diesbezüglich hat der Anmelder in völlig überraschender Weise herausgefunden, daß sich hervorragende Eigenschaften hinsichtlich des FRET-Paares ergeben, wenn der Fluorophor (A) Terbium, insbesondere in Form von Terbium(III), als Metall der Seltenen Erden aufweist.

In diesem Zusammenhang hat es sich gleichermaßen als besonders vorteilhaft erwiesen, wenn der Fluorophor (B) Europium, insbesondere in Form von Europium(III), als Metall der Seltenen Erden aufweist.

Folglich ist es im Rahmen der vorliegenden Erfindung besonders bevorzugt, wenn das im Rahmen der erfindungsgemäßen Verwendung bereitgestellte FRET-Paar einen Fluorophor (A) und einen Fluorophor (B) umfaßt, wobei der Fluorophor (A) Terbium, insbesondere in Form von Terbium(III), als Metall der Seltenen Erden aufweist und der Fluorophor (B) Europium, insbesondere in Form von Europium(III), als Metall der Seltenen Erden aufweist. Ein derartiges FRET-Paar weist exzellente Eigenschaften hinsichtlich des Quench-Verhaltens bei Vorliegen eines Energietransferes, insbesondere Fluoreszenzresonanzenergietransfers, bei geringer Beabstandung der Fluorophore (A) und (B) zueinander sowie ein definiertes Emissionsverhalten mit schmalen Emissionsbanden und verlängerter Emissionsdauer auf, so daß sich ein derartiges FRET-Paar auf Basis von Terbium und Europium in besonderem Maße zur analytischen Verwendung im Bereich der Biochemie bzw. Molekularbiologie eignet.

Was die Ausbildung der Fluorophore des im Rahmen der erfindungsgemäßen Verwendung bereitgestellten FRET-Paares anbelangt, so ist es erfindungsgemäß vorgesehen, daß die Elemente der Seltenen Erden in den Fluorophoren (A) und (B) jeweils komplexiert sind, und zwar jeweils mit mindestens einem Liganden in Form eines Komplex- und/oder Chelatbildners, vorzugsweise mehren Liganden, bevorzugt vier Liganden. Erfindungsgemäß ist es bevorzugt, daß die Elemente der Seltenen Erden in den Fluorophoren (A) und (B), unabhängig voneinander, an mindestens einen Liganden, insbesondere an mehreren Liganden, vorzugsweise an vier Liganden, ionisch, koordinativ und/oder kovalent, insbesondere kovalent, gebunden sind.

In diesem Zusammenhang sollte der Ligand, insbesondere der Komplexund/oder Chelatbildner, mehrdentat, insbesondere bidentat, ausgebildet sein. In diesem Zusammenhang kann der Ligand mehrere koordinative, ionische oder kovalente Bindungen mit dem Kern bzw. dem Zentralatom auf Basis eines Metalls der Seltenen Erden eingehen. Grundsätzlich ist es im Rahmen der vorliegenden Erfindung möglich, daß hinsichtlich der Fluorophore (A) und (B) gleiche oder unterschiedliche Liganden verwendet werden können. Auch innerhalb des jeweiligen Fluorophors (A) oder (B) selbst können gleiche oder unterschiedliche Liganden verwendet werden.

Erfindungsgemäß ist der Ligand Picolinsäure, Picolinate und/oder deren Derivate, insbesondere substituierten Derivate, vorzugsweise Hydroxyderivate.

Um eine Verbindung der Fluorophore mit mindestens einem weiteren Molekül bzw. organischen Rest zu ermöglichen, weist der Ligand insbesondere die Picolinsäure, Picolinate und/oder deren Derivate, Substituenten und/oder funktionelle Gruppen zur vorzugsweise kovalenten Bindung und/oder Kopplung von vorzugsweise organischen Molekülen auf. Diesbezüglich sind die Substituenten und/oder funktionellen Gruppen ausgewählt aus der Gruppe von Amino-, Carboxylat-, Isocyanat-, Thioisocyanat-, Epoxy-, Thiol- und Hydroxygruppen. Auf diese Weise werden somit sozusagen Anknüpfungspunkte für weitere Moleküle bzw. für mindestens einen organischen Rest an die jeweiligen Fluorophore (A) bzw. (B) bereitgestellt. Bei dem organischen Molekül kann es sich beispielsweise, wie nachfolgend noch angeführt, um eine zu einem Zielmolekül bzw. zu einer Zielstruktur komplementären bzw. hiermit interagierbaren Einheit handeln.

Gemäß einer besonders bevorzugten Ausführungsform sollte der Substituent bzw. die funktionelle Gruppe eine Hydroxygruppe sein.

Weiterhin ist es im Rahmen der vorliegenden Erfindung von besonderem Vorteil, wenn der Ligand in Form eines Komplex- und/oder Chelatbildners, Hydroxypicolinsäure und/oder Hydroxypicolinat ist. In diesem Zusammenhang sollte mindestens einer der Liganden durch die vorgenannten Verbindungen gebildet sein, insbesondere wenn eine kovalente Bindung bzw. eine Kopplung von vorzugsweise organischen Molekülen an die Fluorophore (A) und/oder (B) beabsichtigt ist.

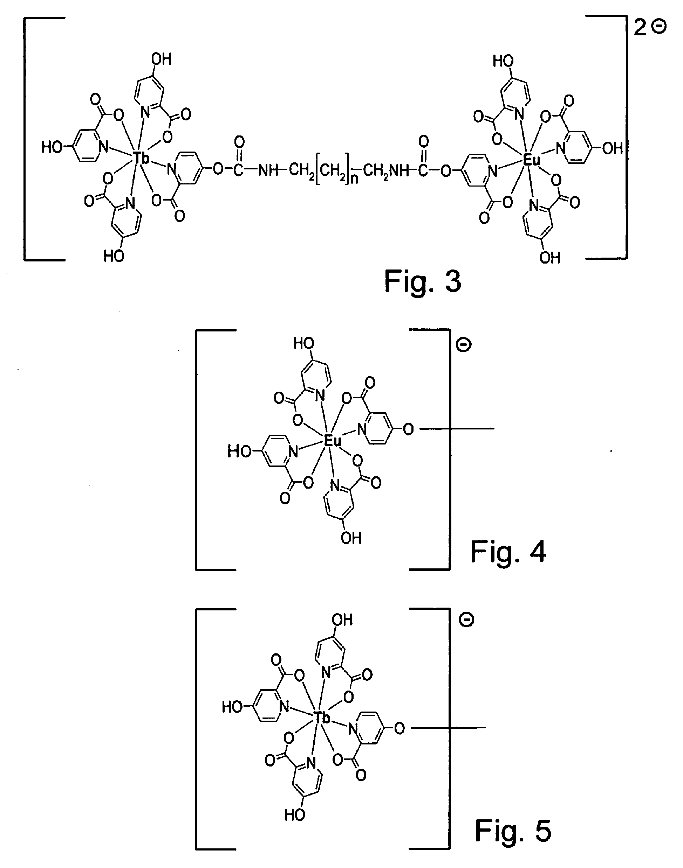

Weiterhin ist es im Rahmen der vorliegenden Erfindung besonders bevorzugt, wenn der Fluorophor (A) ein Organometallkomplex gemäß der Formel von Fig. 5 ist.

Zudem kann es erfindungsgemäß vorgesehen sein, daß der Fluorophor (A) ausgewählt ist aus Tetra(4-hydroxypyridin-2-carboxylato)terbium(III), Tris-(pyridin-2-carboxylato)(4-hydroxypyridin-2-carboxylato)terbium(III), Bis(pyridin-2-carboxylato)-bis(4-hydroxypyridin-2-carboxylato)terbium(III), (Pyridin-2-carboxylato)-tris(4-hydroxypyridin-2-carboxylato)terbium(III) und/oder dessen Derivaten.

Bezüglich der Verbindungen für den Fluorophor (A) können insbesondere auch die entsprechenden Salze, insbesondere Natriumsalze verwendet werden.

Zudem kann der Fluorophor (A) eine Verbindung der allgemeinen Formel

[Tbₓ(Pic)_{y}(Pic-Y)_{z}]^{(4-3x)-}

sein, wobei in der vorbezeichneten Formel
- Tb Terbium(III) ist,
- Pic Picolinat ist,
- Y eine funktionelle Gruppe ist, insbesondere ausgewählt aus der Gruppe von Amino-, Carboxylat-, Isocyanat-, Thioisocyanat-, Epoxy-, Thiol- und Hydroxygruppen, vorzugsweise einer Hydroxygruppe,
- x eine ganze Zahl von 1 bis 4, insbesondere 1 oder 2, vorzugsweise 1, ist und
- y und z jeweils eine ganze Zahl von 0 bis 4 mit y + z = 4 sind.

Was den Fluorophor (B) anbelangt, so kann dieser Fluorophor (B) ein Organometallkomplex gemäß der Formel von Fig. 4 sein

In diesem Zusammenhang ist es erfindungsgemäß besonders bevorzugt, wenn der Fluorophor (A) ein Organometallkomplex gemäß der Formel von Fig. 5 und der Fluorophor (B) ein Organometallkomplex gemäß der Formel von Fig. 4 ist.

Was den Fluorophor (B) weiterhin anbelangt, so kann dieser ausgewählt sein aus Tetra(4-hydroxypyridin-2-carboxylato)europium(III), Tris(pyridin-2-carboxylato)(4-hydroxypyridin-2-carboxylato)europium(III), Bis(pyridin-2-carboxylato)-bis(4-hydroxypyridin-2-carboxylato)europium(III), (Pyridin-2-carboxylato)-tris(4-hydroxypyridin-2-carboxylato)europium(III) und/oder dessen Derivaten.

Auch hinsichtlich der Verbindungen bezüglich des Fluorophors (B) können beispielsweise die entsprechenden Salze, insbesondere Natriumsalze, eingesetzt werden.

In bezug auf den Fluorophor (B) kann auch eine Verbindung der allgemeinen Formel

[Eu_{x'}(Pic')_{y'}(Pic'-Y')_{z'}]^{(4-3x')-}

vorliegen, wobei in der vorbezeichneten Formel
- Eu Europium(III) ist,
- Pic' Picolinat ist,
- Y' eine funktionelle Gruppe ist, insbesondere ausgewählt aus der Gruppe von Amino-, Carboxylat-, Isocyanat-, Thioisocyanat-, Epoxy-, Thiol- und Hydroxygruppen, vorzugsweise einer Hydroxygruppe,
- x' eine ganze Zahl von 1 bis 4, insbesondere 1 oder 2, vorzugsweise 1, ist und
- y' und z' jeweils eine ganze Zahl von 0 bis 4 mit y' + z' = 4 sind.

Im Rahmen der vorliegenden Erfindung ist es vorgesehen, daß insbesondere zur Ausbildung eines FRET-Paares bzw. einer FRET-Sonde der Fluorophor (A) und der Fluorophor (B) über einen zweiwertigen und/oder zweibindigen organischen Rest, insbesondere einen Linker und/oder Spacer, miteinander gekoppelt und/oder verbunden sind.

Diesbezüglich resultiert somit ein Molekül bzw. Molekülkomplex, welches bzw. welcher den Fluorophor (A) und den Fluorophor (B) sowie den Rest umfaßt. Durch die zweckgerichtete Auswahl des organischen Restes und Abstimmung mit den speziellen Fluorophoren (A) und (B) kann somit das FRET-Paar bzw. die FRET-Sonde insofern optimiert bzw. maßgeschneidert werden, als bei geringer Beabstandung der Fluorophore (A) und (B) voneinander ein optimaler Fluoreszenzresonanzenergietransfer im Sinne einer maximalen Quenchung des Donorsignals stattfinden kann und bei zunehmender Beabstandung eine wohldefinierte Änderung des Emissionsspektrums mit scharfen Banden und langen Emissionsdauern resultiert.

Demnach sollte im Rahmen der vorliegenden Erfindung der organische Rest ausgewählt sein derart, daß zwischen dem Fluorophor (A) und dem Fluorophor (B) ein Fluoreszenzresonanzenergietransfer eintreten kann. Dies gilt insbesondere für den Fall einer geringen Beabstandung der Fluorophore (A) und (B) zueinander, beispielsweise wenn keine Wechselwirkung mit einem Zielmolekül bzw. einer Zielstruktur vorliegt. Somit kann durch die gezielte Auswahl des Spacers der Fluoreszenzenergietransfer individuell eingestellt bzw. maßgeschneidert werden, wobei ein kleiner Spacer, d. h. insbesondere ein Molekül mit geringer Kettenlänge, zu einer geringen Beabstandung der Fluorophore (A) und (B) führt, was mit einem hohen Fluoreszenzresonanzenergietransfer einhergeht. Aber auch ein langer Rest bzw. Spacer kann beispielsweise über Faltungs- und/oder Anlagerungs- bzw. Hybridisierungsvorgänge zu einer geringen Beabstandung zwischen (A) und (B) führen, wie es z. B. bei einem Molecular Beacon der Fall ist.

Aufgrund der individuellen Einstellung der Beabstandung der Fluorophore (A) und (B) zueinander durch den organischen Rest kann somit das Emissionsspektrum spezifisch eingestellt werden, insbesondere wobei auch unter FRET Emissionsspektren mit spezifischen Wellenlängen bzw. Emissionsbanden resultieren, was insbesondere im Rahmen der Herstellung bzw. der Verwendung als Farbstoffe von großer Bedeutung ist, da die Farbeigenschaften sozusagen durch die gezielte Einstellung der Beabstandung der Fluorophore (A) und (B) individuell eingestellt werden kann.

Somit kann insgesamt durch die Länge des organischen Restes, dessen Struktur, räumliche Anordnung bzw. Ausbildung die Beabstandung der Fluorophore (A) und (B) zueinander und somit der Fluoreszenzresonanzenergietransfer und damit die Emissionswellenlänge des FRET-Paares eingestellt bzw. maßgeschneidert werden. Die Anbindung bzw. Kopplung des organischen Restes an die Fluorophore kann beispielsweise erfolgen derart, daß der organische Rest an den jeweiligen Substituenten und/oder an die jeweilige funktionelle Gruppe des Komplexbildners und/oder des Liganden, insbesondere wie zuvor definiert, gekoppelt und/oder gebunden werden kann.

Dabei ist es im Rahmen der vorliegenden Erfindung bevorzugt, wenn der organische Rest vorzugsweise kovalent mit dem Fluorophor (A) und/oder dem Fluorophor (B) verbunden ist, insbesondere wobei der organische Rest vorzugsweise kovalent an die funktionellen Gruppen des oder der Liganden des Fluorophors (A) und/oder (B) angebunden und/oder angekoppelt ist und/oder insbesondere wobei der organische Rest zur vorzugsweise kovalenten Bindung und/oder Kopplung an die funktionellen Gruppen des oder der Liganden fähige funktionelle Gruppen aufweist.

Dabei erfolgt die Anbindung bzw. Ankopplung des organischen Restes an die funktionellen Gruppen insbesondere derart, daß die Fluorophore (A) und (B), unabhängig voneinander, vorzugsweise endständig an den organischen Rest und somit gewissermaßen an das jeweilige Ende des organischen Restes gebunden bzw. gekoppelt werden.

In diesem Zusammenhang kann der organische Rest beispielsweise unter Ausbildung von insbesondere in bezug auf den organischen Rest endständigen (Poly-)Urethangruppen kovalent mit dem Fluorophor (A) und/oder dem Fluorophor (B) verbunden sein.

Eine vorzugsweise endständige Bindung bzw. Kopplung der Fluorophore (A) und (B) an einen organischen Rest liegt beispielsweise dann vor, wenn das gemäß der erfindungsgemäßen Verwendung bereitgestellte FRET-Paar als FRET-Sonde nach Art einer Hydrolyse-Sonde oder nach Art eines Molecular Beacon vorliegt bzw. eingesetzt werden soll.

Was den organischen Rest im allgemeinen anbelangt, so kann der organische Rest ein Rest sein, welcher mit einem Zielmolekül und/oder einer Zielstruktur in Wechselwirkung zu treten imstande ist. Der organische Rest kann derart sein, daß es sich um einen mit einem Zielmolekül und/oder einer Zielstruktur interagierbaren Rest handelt.

Im Rahmen der vorliegenden Erfindung kann es vorgesehen sein, daß eine Interaktion und/oder Wechselwirkung des organischen Restes mit dem Zielmolekül und/oder der Zielstruktur zu einer Änderung, insbesondere zu einer Verringerung, des Fluoreszenzresonanzenergietransfers von dem Fluorophor (A) auf den Fluorophor (B) führt.

Dies wird insbesondere dadurch erreicht, daß aufgrund der Interaktion bzw. Wechselwirkung die Beabstandung zwischen dem ersten Fluorophor (A) und dem zweiten Fluorophor (B) vergrößert wird, so daß der Energietransfer zwischen den Fluorophoren abnimmt und somit das Emissionsverhalten geändert wird. Auf diese Weise kann die Interaktion bzw. Wechselwirkung durch Erfassung (einer Änderung) des Emissionsspektrums der Probe nachgewiesen werden.

Was den organischen Rest anbelangt, so kann dieser beispielsweise eine Nukleotidsequenz, insbesondere eine DNA- oder RNA-Sequenz, vorzugsweise ein Oligonukleotid, sein oder diese umfassen.

Dies kann beispielsweise dann der Fall sein, wenn das im Rahmen der erfindungsgemäßen Verwendung bereitgestellte FRET-Paar als Hybridisierungs-Sonde, Hydrolyse-Sonde und/oder als Molecular Beacon eingesetzt wird.

Insbesondere für den Fall, daß das gemäß der erfindungsgemäßen Verwendung bereitgestellte FRET-Paar als Molecular Beacon eingesetzt wird, kann es vorgesehen sein, daß die Nukleotid-Sequenz, insbesondere an deren 5'-Ende und an deren 3'-Ende, komplementäre Nukleotid-Sequenzabschnitte aufweist, insbesondere so daß bei nicht vorhandener Wechselwirkung mit einem Zielmolekül und/oder einer Zielstruktur eine Hybridisierung der komplementären Nukleotid-Sequenzabschnitte resultiert bzw. vorliegt. Durch die komplementären Endabschnitte der Nukleotid-Sequenz resultiert gewissermaßen eine Selbsthybridisierung der Enden der Nukleotid-Sequenz, so daß die zuvor beschriebene und für Molecular Beacons charakteristische Struktur im Sinne eines Stem Loop vorliegt. In diesem Zustand weist das Fluorophor (A) durch seinen geringen Abstand zum Fluorophor (B) keine bzw. eine verminderte Fluoreszenz auf. Im Falle einer Anlagerung der Loop-Region bzw. SchleifenRegion an eine komplementäre DNA-Sequenz als Zielmolekül bzw. Zielstruktur, beispielsweise entstanden während eines PCR-Zyklus, wird der Abstand zwischen dem Fluorophor (A) und dem Fluorophor (B) vergrößert, so daß für diesen Fall bedingt durch einen verringerten Fluoreszenzresonanzenergietransfer eine Fluoreszenz bzw. eine Veränderung der Fluoreszenz in bezug auf den Fluorophor (A) resultiert und dieser bzw. diese mit dem Fachmann an sich bekannten Vorrichtungen bzw. Verfahren beobachtet bzw. detektiert werden kann, wobei die Veränderung des Emissionsspektrums mit einer Zunahme der Donorfluoreszenzen und einer Abnahme der Akzeptorfluoreszenz einhergehen kann.

Wie zuvor angeführt, kann es sich bei dem nach der erfindungsgemäßen Verwendung bereitgestellten FRET-Paar beispielsweise um ein Molecular Beacon (MB) handeln. In diesem Zusammenhang ist es vorgesehen, daß der Fluorophor (A) und der Fluorophor (B) durch insbesondere kovalente Bindung an eine Nukleotidsequenz mit insbesondere an deren 5'-Ende und 3'-Ende komplementären Nukleotidsequenzabschnitten ein Molecular Beacon (MB) ausbilden.

Gleichermaßen ist es im Rahmen der vorliegenden Erfindung jedoch auch möglich, daß der organische Rest ein Peptid und/oder eine Aminosäuresequenz, insbesondere ein Oligopeptid, ist oder dieses bzw. diese umfaßt.

Hierbei kann es sich beispielsweise um ein Enzym oder um eine definierte, auf eine jeweilige Zielstruktur bzw. auf ein Zielmolekül ausgerichtete Aminosäuresequenz handeln. Gleichermaßen kann es sich aber auch um ein Peptid bzw. um eine Aminosäuresequenz handeln, deren räumliche Struktur selbst erfaßt werden soll.

Zudem kann es im Rahmen der vorliegenden Erfindung auch vorgesehen sein, daß der organische Rest ein gesättigter oder ungesättigter, vorzugsweise ein ungesättigter, verzweigter oder linearer Kohlenwasserstoffrest ist oder diesen umfaßt.

In diesem Zusammenhang ist es besonders vorteilhaft, wenn der Kohlenwasserstoffrest eine Kettenlänge von mindestens 2 Kohlenstoffatomen, insbesondere 2 bis 20 Kohlenstoffatomen, vorzugsweise 2 bis 15 Kohlenstoffatomen, bevorzugt 2 bis 12 Kohlenstoffatomen, aufweist.

Auch in diesem Fall sollte, wie zuvor angeführt, die Länge des Kohlenwasserstoffrestes vor dem Hintergrund des einzustellenden Energietransfers, insbesondere Fluoreszenzresonanzenergietransfers, zwischen dem Fluorophor (A) und dem Fluorophor (B) ausgewählt werden.

Gemäß einer erfindungsgemäß besonders bevorzugten Ausführungsform bilden der Fluorophor (A) und der Fluorophor (B) zusammen mit einem organischen Rest den Komplex gemäß der Formel von Fig. 3 aus, wobei in der vorbezeichneten Formel n eine ganze Zahl im Bereich von 1 bis 20, insbesondere 2 bis 15, vorzugsweise 2 bis 12, darstellt.

Gemäß den Formeln von Fig. 3 bis 5 wird der Fluorophor (A) durch einen Terbium-Komplex mit vier Hydroxypicolinaten sowie der Fluorophor (B) durch einen Europium-Komplex mit vier Hydroxypicolinaten gebildet. Die Fluorophore sind durch einen Kohlenwasserstoffrest an endständigen Urethanbindungen miteinander verbunden.

Was den organischen Rest anbelangt, so kann dieser auch ein Mischmolekül darstellen, welches eine Nukleotid-Sequenz und/oder eine AminosäureSequenz und/oder einen Kohlenwasserstoffrest enthält.

Die vorliegende Erfindung wird anhand der Figurendarstellung weiter und rein exemplarisch veranschaulicht, ohne die Erfindung jedoch hierauf zu beschränken. Es zeigt:

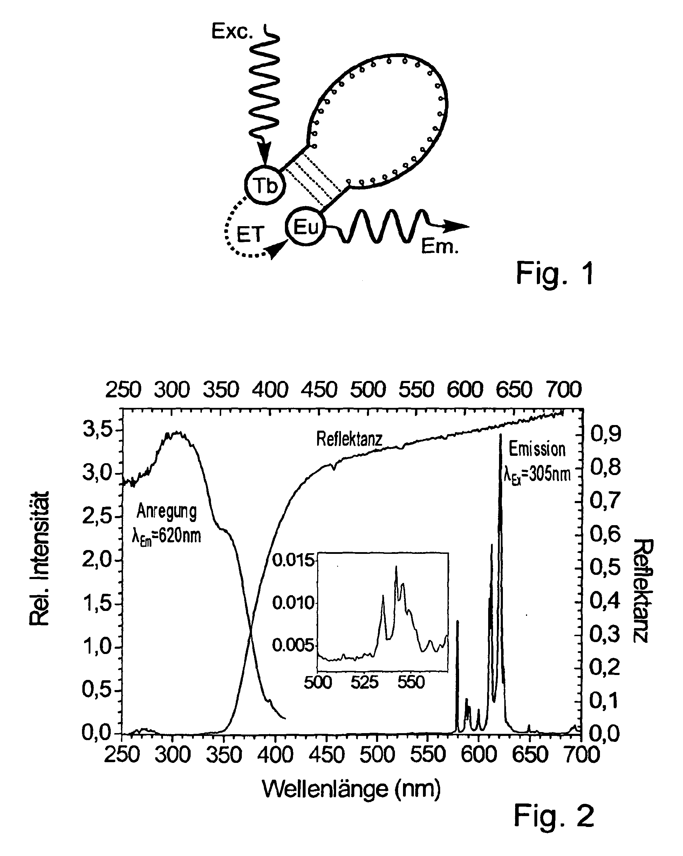

- Fig. 1: einen erfindungsgemäßen FRET-Komplex, welcher einen Terbium-Komplex (Tb) als Donorfluorophor und einen Europium-Komplex (Eu) als Akzeptorfluorophor aufweist. Die beiden Fluorophore sind über einen organischen Rest ("Spacer" bzw. "Linker") miteinander verbunden, wobei es sich hierbei insbesondere um eine Nukleotid-Sequenz handeln kann, welche an deren Enden zueinander komplementäre Nukleotide aufweist, die hybridisiert sind. Der nicht hybridisierte Teil der Nukleotid-Sequenz bildet eine Schleifenregion aus. Der in Fig. 1 dargestellte Komplex stellt insbesondere ein Molecular Beacon (MB) dar, welches in einem geschlossenen Zustand vorliegt, wobei im vorliegenden Fall bei Einstrahlung einer Anregungsenergie (Exc.) ein insbesondere strahlungsfreier Energietransfer (ET) des Terbium-Komplexes auf den Europium-Komplex, einhergehend mit einer spezifischen Emission (Em.) durch den Europium-Komplex, eintritt.
- Fig. 2: ein optisches Spektrum des in Fig. 3 dargestellten erfindungsgemäßen FRET-Komplexes. Aufgrund des effektiven Energietransfers des Tb-Komplexes auf den Eu-Komplex liegt eine signifikant verringerte bzw. nur sehr geringe Emission durch den Tb-Komplex vor.
- Fig. 3: einen erfindungsgemäßen FRET-Komplex auf Basis eines Tb-Komplexes als Fluorophor (A) und eines Europium-Komplexes als Fluorophor (B), wobei die Komplexe über einen organischen Rest in Form einer linearen Kohlenwasserstoffkette mittels Urethanbrücken verbunden sind.
- Fig. 4: einen im Rahmen der vorliegenden Erfindung eingesetzten Europium-Komplex als Fluorophor (B), welcher vier Hydroxypicolinate als Liganden aufweist.
- Fig. 5: einen im Rahmen der vorliegenden Erfindung eingesetzten Terbium-Komplex als Fluorophor (A), welcher vier Hydroxypicolinate als Liganden aufweist.

Weiterhin betrifft die vorliegende Erfindung - gemäß einem zweiten Aspekt der vorliegenden Erfindung einen Fluoreszenzresonanzenergietransfer-Komplex (FRET-Komplex), aufweisend mindestens einen ersten Fluorophor (A) als Donor-Fluorophor und mindestens einen zweiten Fluorophor (B) als Akzeptor-Fluorophor. Der erfindungsgemäße FRET-Komplex zeichnet sich dadurch aus, daß der erste Fluorophor (A) und der zweite Fluorophor (B), unabhängig voneinander, jeweils auf Basis eines Organometallkomplexes von Elementen der Seltenen Erden ausgebildet sind, wobei die Fluorophore (A) und (B) voneinander verschiedene Elemente der Seltenen Erden, ausgewählt aus der Gruppe von Europium und Terbium, aufweisen. Weiterhin sind die Elemente der Seltenen Erden in den Flurophoren (A) und (B) jeweils mit mindestens einem Liganden in Form eines Komplex- und/oder Chelatbildners komplexiert, wobei der Ligand Picolinsäure, Picolinat und/oder deren Derivate ist und wobei der Ligand Substituenten und/oder funktionelle Gruppen zur vorzugsweise kovalenten Bindung und/oder Kopplung von vorzugsweise organischen Molekülen aufweist, wobei die Substituenten und/oder funktionellen Gruppen ausgewählt sind aus der Gruppe von Amino-, Carboxylat-, Isocyanat-, Thioisocyanat-, Epoxy-, Thiol- und Hydroxygruppen. Zudem sind der Fluorophor (A) und der Fluorophor (B) über einen zweiwertigen und/oder zweibindigen organischen Rest, insbesondere einen Linker und/oder Spacer, miteinander gekoppelt und/oder verbunden.

Gemäß einer bevorzugten Ausführungsform der vorliegenden Erfindung kann der erfindungsgemäße FRET-Komplex der allgemeinen Formel

DF-S-AF

entsprechen, wobei in der vorbezeichneten Formel
- DF einen Donorfluorophor, insbesondere den Fluorophor (A), bezeichnet,
- AF einen Akzeptorfluorophor, insbesondere den Fluorophor (B), bezeichnet und
- S einen insbesondere zweiwertigen und/oder zweibindigen organischen Rest, insbesondere einen Linker und/oder Spacer, bezeichnet.

Für weitere Ausführungen in bezug auf den erfindungsgemäßen FRET-Komplex kann auf die Ausführungen zu der oben angeführten erfindungsgemäßen Verwendung verwiesen werden, welche in bezug auf den erfindungsgemäßen FRET-Komplex gleichermaßen gelten.

Der erfindungsgemäße FRET-Komplex kann beispielsweise im Hinblick auf analytische Verfahren beispielsweise in Form einer Hybridisierungs-Sonde, einer Hydrolyse-Sonde oder eines Molecular Beacon ausgebildet sein.

Insgesamt kann es sich bei den FRET-Komplexen nach der Erfindung um die bereits zuvor beschriebenen Hybridisierungssonden, Hydrolysesonden und Molecular Beacons handeln. In diesem Zusammenhang können die erfindungsgemäßen FRET-Komplexe über die spezielle Auswahl der Fluorophore (A) und (B) und des organischen Restes hinsichtlich ihrer Energietransfereigenschaften bzw. -parameter eingestellt werden. Die so eingestellten Energietransfereigenschaften können zur Kalibrierung intramolekularer Entfernungen bzw. Beabstandungen in hybridisierten Nukleotidmolekülen insbesondere in Form von Molecular Beacons verwendet werden, welche mit den speziellen Fluorophoren (A) und (B) an den jeweiligen Enden ausgerüstet sind, wobei der organische Rest in Form eines Oligonukleotids somit als Spacer bzw. Linker fungiert. Durch die biochemische Induktion mittels einer geeigneten Zielsequenz bzw. Zielstruktur öffnet sich die schleifenförmig geschlossene Nukleotidsequenz, so daß eine veränderte Emissionsantwort aufgrund eines geänderten Energietransfers resultiert, wenn die Enden der Schleife separiert bzw. getrennt werden.

Die erfindungsgemäßen FRET-Komplexe weisen insbesondere wohldefinierte Emissionsspektren auf, wobei die Emissionsdauer nach Anregung durch ein eingetragenes Signal im Millisekunden-Bereich liegt und somit eine zusätzliche Diskriminierung gegenüber einer unspezifischen Autofluoreszenz möglich ist. Diese beiden Faktoren führen dazu, daß auf aufwendige Laser- und Auswertesysteme zur Analyse der Meßsignale verzichtet werden kann. Zudem liegt bei kleiner Beabstandung der Fluorophore eine optimale Quenchung vor, was die Signalqualität weiter verbessert.

Was den erfindungsgemäßen FRET-Komplex weiterhin anbelangt, so kann im Rahmen der entsprechenden Verwendung als Biosensoren eine sehr spezifische und aussagekräftige Realtime-Erfassung, beispielsweise im Rahmen der Amplifikation von Zielnukleinsäuren, mit einer niedriger Nachweisgrenze, insbesondere im Rahmen der Realtime-PCR in einem kompakt und einfach gestalteten Thermocycler, durchgeführt werden.

Der Einsatz des erfindungsgemäßen FRET-Komplexes ist nicht auf die Kopplung an Nukleinsäuren beschränkt. So ist es ebenso möglich, daß der erfindungsgemäße FRET-Komplex in Form eines Proteins als organischer Rest, welcher die Fluorophore (A) und (B) verbindet, vorliegt und im Falle einer Spaltung des Proteins oder auch aufgrund einer Konformationsänderung, beispielsweise im Falle der Bindung an einen Liganden, eine Veränderung der räumlichen Beabstandung der Fluorophore (A) und (B) resultiert, so daß eine Änderung des Emissionsspektrums vorliegt. Insbesondere wird auch in diesem Fall die Fluoreszenzemission des Donorfluorophors und somit des Fluorophors (A) nachweisbar. Somit kann der erfindungsgemäße FRET-Komplex auch in der Protein- oder Peptidanalytik eingesetzt werden.

Darüber hinaus können die erfindungsgemäßen FRET-Komplexe auch in isothermalen Amplifikationsverfahren, wie *Nucleic Acid Sequence Based Amplification* (NASBA) eingesetzt werden. Die erfindungsgemäßen FRET-Komplexe können auch in der Array-Technologie verwendet werden. Zudem ist eine Anlagerung bzw. Anbindung der erfindungsgemäßen FRET-Komplexe an ein festes Substrat, beispielsweise zur Verwendung in Biochips bzw. Biosensoren, möglich. Zudem kommt gleichermaßen eine Verwendung der erfindungsgemäßen FRET-Komplexe in Form von Aptameren in Betracht. Insgesamt eröffnet sich somit in bezug auf die erfindungsgemäßen FRET-Komplexe das sehr große Anwendungsfeld der medizinischen, molekularbiologischen bzw. biochemischen Analytik und Diagnostik.

Für weitere Ausführungsformen betreffend den dritten Aspekt der vorliegenden Erfindung kann auf die obigen Ausführungen zu dem ersten und zweiten Aspekt der vorliegenden Erfindung verwiesen werden, welche entsprechend gelten.

Weiterer Gegenstand der vorliegenden Erfindung - gemäß einem dritten Aspekt der vorliegenden Erfindung - ist die erfindungsgemäße Verwendung des zuvor definierten FRET-Komplexes nach der Erfindung zur Detektion eines Ereignisses in einer Probe.

Der Begriff "Ereignis", wie er im Rahmen der vorliegenden Erfindung verwendet wird, ist sehr breit zu verstehen. So kann es sich beispielsweise bei dem Ereignis um eine Interaktion und/oder Wechselwirkung und/oder Hybridisierung des FRET-Komplexes mit einer DNA-Sequenz oder RNA-Sequenz oder einem Oligonukleotid handeln, beispielsweise im Rahmen eines Nukleinsäurenachweises. In diesem Falle weist der organische Rest des erfindungsgemäßen FRET-Komplexes vorzugsweise eine Nukleotid-Sequenz auf oder besteht aus dieser. Gleichermaßen umfaßt der Begriff "Ereignis" auch eine Interaktion und/oder Wechselwirkung und/oder Anlagerung des erfindungsgemäßen FRET-Komplexes an ein (Poly-)Peptid bzw. Enzym. Gleichermaßen kann das Ereignis auch die Spaltung oder aber die Bindung eines Peptids an einen Liganden sein. In diesem Fall sollte der organische Rest des erfindungsgemäßen FRET-Komplexes vorzugsweise eine AminosäureSequenz bzw. ein Oligo- oder Polypeptid sein oder diese bzw. dieses umfassen. Gleichermaßen kann das Ereignis auch eine räumliche Strukturänderung, beispielsweise eine Konfigurations- oder Konformationsänderung, eines Proteins oder dergleichen sein. Das Ereignis stellt somit im allgemeinen gewissermaßen eine Aktivität in einer Meßprobe dar, welche insbesondere zu einer Veränderung der räumlichen Anordnung bzw. der Beabstandung des Fluorophors (A) und des Fluorophors (B) zueinander führt, insbesondere wobei infolge bzw. aufgrund dieser Änderung eine vorzugsweise diskriminierende Erfassung eines veränderten Meßsignals, insbesondere eine Veränderung in dem Emissionsspektrum des FRET-Komplexes vorliegt, welche z. B. auf das zunehmende Signal des als Donor fungierenden Fluorophors (A) und das abnehmende Signal des Akzeptors - bedingt durch die Vergrößerung der räumlichen Beabstandung der Fluorophore (A) und (B) mit Verringerung des Fluoreszenzresonanzenergietransfers - zurückgeführt werden kann.

Was die erfindungsgemäße Verwendung gemäß diesem Aspekt der vorliegenden Erfindung anbelangt, so kann der FRET-Komplex bei Eintritt des Ereignisses und/oder infolge des Ereignisses insbesondere unter Einfluß und/oder Einwirkung und/oder Eintrag einer Anregungsenergie eine Änderung seines Emissionsspektrums erfahren. Zudem kann der FRET-Komplex bei Eintritt des Ereignisses und/oder infolge des Ereignisses insbesondere unter Einfluß und/oder Einwirkung und/oder Eintrag einer Anregungsenergie ein detektierbares Signal emittieren.

Erfindungsgemäß kann es somit vorgesehen sein, daß die relative Anordnung der Fluorophore (A) und (B) zueinander durch oder infolge des Ereignisses verändert wird insbesondere derart, daß eine Änderung des Emissionsspektrums des FRET-Komplexes resultiert und/oder daß ein detektierbares Signal emittiert wird.

Erfindungsgemäß kann es zudem vorgesehen sein, daß die Änderung des Emissionsspektrums und/oder des detektierbaren Signals in zeitlicher Abfolge verzögert zum Eintritt des Ereignisses erfolgt und/oder daß die Änderung des Emissionsspektrums und/oder des detektierbaren Signals in zeitlicher Abfolge nach dem Eintritt des Ereignisses erfolgt.

Weiterhin kann die Erfassung der Änderung des Emissionsspektrums und/oder des detektierbaren Signals durch eine zeitaufgelöste Fluoreszenzmessung erfolgen. Diesbezüglich sollte die Änderung des Emissionsspektrums bzw. das detektierbare Signal von der Autofluoreszenz unterscheidbar sein.

Diesbezüglich ist es vorteilhaft, wenn das detektierbare Signal des FRET-Komplexes im Vergleich zur Autofluoreszenz der Probe um einen Faktor 2, insbesondere um einen Faktor 5, vorzugsweise um einen Faktor 10, bevorzugt um einen Faktor 100, verlängert ist.

Schließlich kann, wie zuvor angeführt, das Ereignis eine Wechselwirkung und/oder Interaktion und/oder Hybridisierung des FRET-Komplexes mit einem Zielmolekül und/oder einer Zielstruktur, insbesondere einer Nukleotidsequenz, vorzugsweise einer DNA- oder RNA-Sequenz, oder einem Peptid und/oder einer Aminosäuresequenz sein. Zudem kann das Ereignis eine räumliche Änderung, insbesondere eine Konfigurations- und/oder Konformationsänderung, des FRET-Komplexes sein.

Weiterer Gegenstand der vorliegenden Erfindung - gemäß einem vierten Aspekt der vorliegenden Erfindung - ist die erfindungsgemäße Verwendung des FRET-Komplexes nach der Erfindung, wie zuvor definiert, zur Interaktion mit einem Zielmolekül und/oder einer Zielstruktur und/oder zur Detektion und/oder Identifizierung und/oder zum Nachweis eines Zielmoleküls und/oder einer Zielstruktur, insbesondere durch Wechselwirkung des FRET-Komplexes einerseits mit dem Zielmolekül und/oder der Zielstruktur andererseits.

Was das Zielmolekül bzw. die Zielstruktur anbelangt, so kann dieses bzw. diese eine Nukleotidsequenz, vorzugsweise eine DNA- oder RNA-Sequenz, ein (Poly-)Peptid und/oder eine Aminosäuresequenz, insbesondere ein Enzym, oder ein Antikörper sein.

Gleichermaßen ist es im Rahmen der vorliegenden Erfindung auch möglich, Viren, Mikroorganismen, insbesondere Bakterien, auf Basis des erfindungsgemäßen FRET-Komplexes zu detektieren bzw. zu erfassen. In diesem Zusammenhang sollte der erfindungsgemäße FRET-Komplex insbesondere hinsichtlich des entsprechenden organischen Restes derart ausgebildet sein, daß eine Interaktion mit Viren, Mikroorganismen, insbesondere Bakterien, Antikörpern und dergleichen bzw. mit bestimmten Zielstrukturen der vorgenannten biologischen Systeme erfolgen kann.

Beispielsweise kann der erfindungsgemäße FRET-Komplex zur Identifizierung bzw. Quantifizierung von Mikroorganismen, insbesondere von Mikroorganismen in Lebensmitteln, verwendet bzw. eingesetzt werden, wobei beispielsweise zunächst eine Anreicherung der Mikroorganismen erfolgen kann. Dies kann beispielsweise und in nichtbeschränkender Weise mit einem mit Liganden beschichteten Träger erfolgen, wobei der Ligand für die zu identifizierenden bzw. quantifizierenden Mikroorganismen spezifisch ist. Zudem kann die Anreicherung auch über durch Antikörper beschichtete Mikropartikel erfolgen. Nachfolgend kann nach gegebenenfalls vorgenommener Lyse und Aufreinigung der DNA der Mikroorganismen eine Amplifikation der entsprechenden Target-Nukleinsäuren unter Verwendung der erfindungsgemäßen FRET-Komplexe bzw. FRET-Sonden erfolgen.

Die vorliegende Erfindung umfaßt gleichermaßen ein Verfahren zur Erfassung bzw. Detektion von Zieloligonukleotiden in einer Probenumgebung, welches die nachfolgenden Schritte umfaßt: In einem ersten Schritt erfolgt eine Inkubation einer Zielnukleinsäure unter Verwendung des erfindungsgemäßen FRET-Komplexes mit anschließender Bestrahlung des Inkubationsansatzes und Erfassung der Fluoreszenzemission, insbesondere mittels zeitaufgelöster Fluoreszenzmessung. In diesem Zusammenhang kann zur Amplifikation der Zielnukleinsäuren beispielsweise eine Realtime-PCR eingesetzt werden. Gleichermaßen kann diesbezüglich die Zielnukleinsäure oder der FRET-Komplex nach der Erfindung als FRET-Sonde auf einem Träger gebunden sein.

Schließlich betrifft die vorliegende Erfindung - gemäß einem fünften Aspekt der vorliegenden Erfindung - die Verwendung des erfindungsgemäßen FRET-Komplexes, wie zuvor definiert, als Farbstoff, insbesondere Fluoreszenzfarbstoff, insbesondere für Markierungszwecke.

Denn aufgrund der speziellen Struktur, einhergehend mit den speziellen Fluorophoren auf Basis von Metallen der Seltenen Erden, können in spezifischer Weise durch die Auswahl der Fluorophore und die Einstellung der Beabstandung der Fluorophore spezifische Farbgebungen hervorgerufen werden, so daß sich die erfindungsgemäßen FRET-Komplexe auch in hervorragender Weise zur Verwendung als Farbstoffe eignen.

Durch die scharfen und eng begrenzten Emissionsbanden sind in bezug auf Farbstoffe definierte Farbgebungen in einem eng begrenzten Spektrum möglich.

Insgesamt kann das der vorliegenden Erfindung zugrundeliegende Prinzip der Ausnutzung des Energietransfers bzw. Fluoreszenzresonanzenergietransfers zwischen Komplexen von Terbium einerseits und Komplexen von Europium andererseits als ein wertvolles Mittel im Rahmen von Strukturstudien, einer Emissions(fein)abstimmung sowie der Verbesserung der Eigenschaften von Biosensoren bzw. Farbstoffkomplexen genutzt werden. Hierin liegt ein klarer Vorteil der vorliegenden Erfindung.

Weiterhin betrifft die vorliegende Erfindung - gemäß einem sechsten Aspekt der vorliegenden Erfindung - die Verwendung des erfindungsgemäßen FRET-Komplexes, wie zuvor definiert, in oder als Biosensoren oder aber in oder als Sonden, insbesondere FRET-Sonden.

Für weitere Ausführungsformen betreffend den vierten bis sechsten Aspekt der vorliegenden Erfindung kann auf die obigen Ausführungen zu den übrigen Aspekten der vorliegenden Erfindung verwiesen werden, welche entsprechend gelten.

Weitere Ausgestaltungen, Abwandlungen und Variationen der vorliegenden Erfindung sind für den Fachmann beim Lesen der Beschreibung ohne weiteres erkennbar und realisierbar, ohne daß er dabei den Rahmen der vorliegenden Erfindung verläßt.

Die vorliegende Erfindung wird anhand der nachfolgenden Ausführungsbeispiele veranschaulicht, welche die vorliegende Erfindung jedoch keinesfalls beschränken.

### AUSFÜHRUNGSBEISPIELE:

### Ligandenmodifizierung:

3-Aminopicolinsäure oder 3-Hydroxypicolinsäure wird in destilliertem Wasser aufgelöst, mit der entsprechende Menge 1 M NaOH-Lösung neutralisiert und als 3-Aminopicolinsäurenatriumsalz bzw. 3-Hydroxypicolinsäurenatriumsalz auskristallisiert.

0,3222 g 3-Aminopicolinsäurenatriumsalz oder 3-Hydroxypicolinsäurenatriumsalz (2 mmol) werden im Vakuum (2 • 10⁻² mbar) bei 110 °C innerhalb 3 Stunden getrocknet, in circa 30 ml getrocknetem DMF aufgelöst und z. B. mit 0,162 ml Hexamethylendiisocyanat (HDI bzw. OCN-(CH₂)₆-CNO) oder z. B. 0,269 ml Dodecamethylendiisocyanat (DMDI bzw. OCN-(CH₂)₁₂-CNO), (1 mmol) unter Schutzgasatmosphäre versetzt. Die Reaktion wird, ebenfalls unter Schutzgas, bei 90 °C innerhalb von 24 Stunden bzw. 70 Stunden bei der Umsetzung des Natrium-3-hydroxypicolinats durchgeführt. DMF wird im Vakuum bei 90 °C entfernt.

Die Produkte haben die Zusammensetzungen NaOOC-C₅H₄N-NH-CO-NH-(CH₂)ₙ-NH-CO-NH-C₅H₄N-COONa (n = 6 für das Produkt mit HDI und n = 12 für das Produkt mit DMDI) bei der Umsetzung des Natrium 3-Aminopicolinates und NaOOC-C₅H₄N-O-CO-NH-(CH₂)ₙ-NH-CO-O-C₅H₄N-COONa bei der Umsetzung des Natrium-3-hydroxypicolinats.

Bei der Vergleichsmodifikation der Liganden mit Monoisocyanaten (Phenylisocyanat bzw. Butylisocyanat) und Butylisothiocyanat werden für die gleiche Reaktion entsprechend 2 mmol des Isocyanatreagenzes angesetzt.

### Synthese der Komplexverbindungen:

### Tetrapicolinatkomelexe:

0,4350 g Picolinsäurenatriumsalz (3 mmol) und 0,1600 g 3-Aminopicolinsäurenatriumsalz (oder 3-Hydroxypicolinsäurenatriumsalz) (1 mmol) werden in ca. 30 ml DMSO unter Erwärmen aufgelöst (Bei der Synthese der Komplexe mit nur einer Art des Liganden werden entsprechend 4 mmol entsprechendes Natriumsalzes angesetzt). 0,3664 g EuCl₃ • 6H_{2O} (oder aber 0,3730 g TbCl₃ • 6 H₂O oder aber 0,1832 g EuCl₃ • 6 H_{2O} und 0,1865 g TbCl₃ • 6 H₂O für die Mischkomplexe) (1 mmol) werden in wenig DMSO gelöst und mit der Ligandenlösung versetzt. Die Mischung wird innerhalb von 2 Stunden bei 90 °C reagieren gelassen, dann wird DMSO bei der gleichen Temperatur im Vakuum abdestilliert. Das so erhaltene beigefarbene Pulver wird in THF unter Rückfluß 1 Stunde lang gekocht, filtriert und im Vakuum bei 80 °C eine Stunde lang getrocknet.

Bei der Synthese der Komplexverbindungen mit den modifizierten Liganden wird statt 1 mmol des 3-Aminopicolinsäurenatriumsalzes entsprechend 0,5 mmol des mit dem Diisocyanat (oder 1 mmol des mit dem Isocyanat oder Isothiocyanat) modifizierten Liganden angesetzt.

Wegen den Schwerlöslichkeit des DMDI-modifizierten Liganden wird die Modifikation der Isocyanate mit den Komplexen der Seltenen Erden selbst durchgeführt: 0,35 mmol Komplex NaEuₓTb₍₁₋ₓ₎(Pic)₃(Pic-Y) (x = 0 bis 1, Pic = Picolinat und Pic-Y = 3-Amino- oder 3-Hydroxypicolinat) werden im Vakuum (2 • 10⁻² mbar) bei 110 °C innerhalb von 3 Stunden getrocknet, in circa 20 ml getrocknetem DMSO aufgelöst und mit 0,094 ml DMDI (0,088 mmol) unter Schutzatmosphäre versetzt. Die Reaktion wird unter Argon bei 90 °C innerhalb von 24 Stunden (bzw. 70 Stunden bei der Umsetzung der 3-Hydroxypicolinate) durchgeführt. DMSO wird bei der gleichen Temperatur im Vakuum abdestilliert. Das nun erhaltene beigefarbene Pulver wird in THF unter Rückfluß 1 Stunde lang gekocht, filtriert und im Vakuum bei 80 °C eine Stunde lang getrocknet.

### Tripicolinatkomplexe:

0,2900 g Picolinsäurenatriumsalz (2 mmol) und 0,1600 g 3-Aminopicolinsäurenatriumsalz (oder 3-Hydroxypicolinsäurenatriumsalz) (1 mol) werden in ca. 30 ml DMF unter Erwärmen aufgelöst (Bei der Synthese der Komplexe mit nur einer Art des Liganden werden 3 mmol des entsprechenden Natriumsalzes angesetzt.). 0,3664 g EuCl₃ • 6 H₂O (oder aber 0,3730 g TbCl₃ • 6 H₂O oder aber 0,1832 g EuCl₃ • 6 H₂O und 0,1865 g TbCl • 6 H₂O für die Mischkomplexe) (1 mol) werden in wenig DMF aufgelöst und mit der Ligandenlösung versetzt. Der entstehende Niederschlag wird filtriert, mit Ethanol gewaschen und im Vakuum bei 50 °C eine Stunde lang getrocknet.

Bei der Synthese der Komplexverbindungen mit dem modifizierten Liganden werden statt 1 mmol des 3-Aminopicolinsäurenatriumsalzes entsprechend 0,5 mmol des mit dem Diisocyanat (oder 1 mmol mit dem Isocyanat oder Isothiocyanat) modifizierten Liganden angesetzt.

Bei der Synthese der Komplexverbindungen mit ausschließlich modifizierten Liganden, z. B. EU₂[NaOOC-C₅H₄N-O-CO-NH-(CH₂)ₙ-NH-CO-O-C₅H₄N-COO]₃, entsteht der Niederschlag erst beim Erwärmen auf 90 bis 100 °C.

### Markierung von Isocyanat-modifizierter DNA:

Der Komplex NaEuₓTb₍₁₋ₓ₎(Pic)₃(Pic-Y) (z. B. x = 0 für die Markierung mit nur grün leuchtendem Komplex; x = 1 für die Markierung mit nur rot leuchtendem Komplex; x = 0,5 für die Markierung mit grün und rot leuchtenden Komplexen; Pic = Picolinat; Pic-Y = 3-Amino- oder 3-Hydroxypicolinat) wird im Vakuum (2 • 10⁻² mbar) bei 110 °C innerhalb von 3 Stunden getrocknet, in DMSO gelöst und mit der entsprechenden Menge der DNA-Lösung in getrocknetem DMSO (molares Verhältnis Pic-Y : NCO = 1 : 1) unter Schutzatmosphäre versetzt. Die Reaktion wird unter Argon bei 90 °C innerhalb von 24 Stunden (bzw. 70 Stunden bei der Umsetzung der 3-Hydroxypicolinate) durchgeführt. Falls erforderlich kann das DMSO bei der gleichen Temperatur im Vakuum abdestilliert werden.

### Markierung von Isothiocyanat-modifizierter DNA:

Der Komplex NaEuₓTb₍₁₋ₓ₎(PiC)₃(Pic-Y) (z. B. x = 0 für die Markierung mit nur grün leuchtendem Komplex; x = 1 für die Markierung mit nur rot leuchtendem Komplex; x = 0,5 fiir die Markierung mit grün und rot leuchtenden Komplexen; Pic = Picolinat; Pic-Y = 3-Amino- oder 3-Hydroxypicolinat) wird in DMSO gelöst und mit der entsprechenden Menge der DNA-Lösung in Wasser, DMSO oder DMSO/Wasser-Mischung (molares Verhältnis Pic-Y : NCS = 1 : 1) versetzt. Die Reaktion wird innerhalb von 24 Stunden (bzw. 70 Stunden bei der Umsetzung der 3-Hydroxypicolinate) durchgeführt. Falls erforderlich kann das DMSO bzw. das Wasser bei der gleichen Temperatur im Vakuum abdestilliert werden.

## Patentansprüche

1. Verwendung von mindestens zwei voneinander verschiedenen Fluorophoren zur Ausbildung eines Fluoreszenzresonanzenergietransfer-Paares (FRET-Paares), wobei innerhalb des FRET-Paares mindestens ein erster Fluorophor (A) als Donorfluorophor und mindestens ein zweiter Fluorophor (B) als Akzeptorfluorophor dient,
wobei der erste Fluorophor (A) und der zweite Fluorophor (B), unabhängig voneinander, jeweils auf Basis eines Organometallkomplexes von Elementen der Seltenen Erden ausgebildet sind, wobei die Fluorophore (A) und (B) voneinander verschiedene Elemente der Seltenen Erden, ausgewählt aus der Gruppe von Europium und Terbium, aufweisen,
wobei die Elemente der Seltenen Erden in den Fluorophoren (A) und (B) jeweils mit mindestens einem Liganden in Form eines Komplex- und/oder Chelatbildners komplexiert sind, wobei der Ligand Picolinsäure, Picolinate und/oder deren Derivate ist und wobei der Ligand Substituenten und/oder funktionelle Gruppen zur vorzugsweise kovalenten Bindung und/oder Kopplung von vorzugsweise organischen Molekülen aufweist, wobei die Substituenten und/oder funktionellen Gruppen ausgewählt sind aus der Gruppe von Amino-, Carboxy-lat-, Isocyanat-, Thioisocyanat-, Epoxy-, Thiol- und Hydroxygruppen, und
wobei der Fluorophor (A) und der Fluorophor (B) über einen zweiwertigen und/oder zweibindigen organischen Rest miteinander gekoppelt und/oder verbunden sind.

2. Verwendung nach Anspruch 1, **dadurch gekennzeichnet,**
**daß** der Fluorophor (A) Terbium in Form von Terbium(III) als Metall der Seltenen Erden aufweist und/oder
**daß** der Fluorophor (B) Europium in Form von Europium(III) als Metall der Seltenen Erden aufweist.

3. Verwendung nach Anspruch 1 oder 2, **dadurch gekennzeichnet,**
**daß** die Elemente der Seltenen Erden in den Fluorophoren (A) und (B) mehren Liganden, bevorzugt vier Liganden, komplexiert sind und/oder daß die Elemente der Seltenen Erden in den Fluorophoren (A) und (B), unabhängig voneinander, an dem mindestens einen Liganden, insbesondere an mehreren Liganden, vorzugsweise an vier Liganden, ionisch, koordinativ und/oder kovalent, insbesondere kovalent, gebunden sind und/oder
**daß** der Ligand mehrdentat, insbesondere bidentat, ausgebildet ist und/oder
**daß** der Ligand, insbesondere Komplexbildner und/oder Chelatbildner, Picolinsäure, Picolinate und/oder deren Derivate, insbesondere substituierte Derivate, vorzugsweise Hydroxyderivate, ist und/oder
**daß** der Ligand Hydroxypicolinsäure und/oder Hydroxypicolinat ist.

4. Verwendung nach einem der vorangehenden Ansprüche, **dadurch gekennzeichnet,**
**daß** der Fluorophor (A) ein Organometallkomplex gemäß der Formel 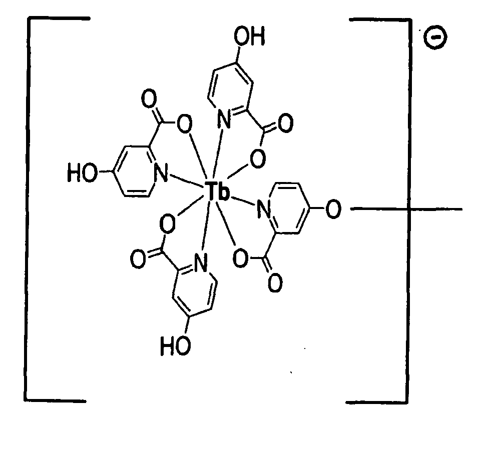 ist und/oder
**daß** der Fluorophor (A) ausgewählt ist aus Tetra(4-hydroxypyridin-2-carboxylato)terbium(III), Tris(pyridin-2-carboxylato)(4-hydroxypyridin-2-carboxylato)-terbium(III), Bis(pyridin-2-carboxylato)-bis(4-hydroxypyridin-2-carboxylato)terbium(III), (Pyridin-2-carboxylato)-tris(4-hydroxypyridin-2-carboxylato)-terbium(III) und/oder dessen Derivaten und/oder daß der Fluorophor (A) eine Verbindung der allgemeinen Formel
[Tbₓ(Pic)_{y}(Pic-Y)_{z}]^{(4-3x)-}
ist, wobei in der vorbezeichneten Formel
• Tb Terbium(III) ist,
• Pic Picolinat ist,
• Y eine funktionelle Gruppe ist, insbesondere ausgewählt aus der Gruppe von Amino-, Carboxylat-, Isocyanat-, Thioisocyanat-, Epoxy-, Thiol- und Hydroxygruppen, vorzugsweise einer Hydroxygruppe,
• x eine ganze Zahl von 1 bis 4, insbesondere 1 oder 2, vorzugsweise 1, ist und
• y und z jeweils eine ganze Zahl von 0 bis 4 mit y + z = 4 sind.

5. Verwendung nach einem der vorangehenden Ansprüche, **dadurch gekennzeichnet,**
**daß** der Fluorophor (B) ein Organometallkomplex gemäß der Formel 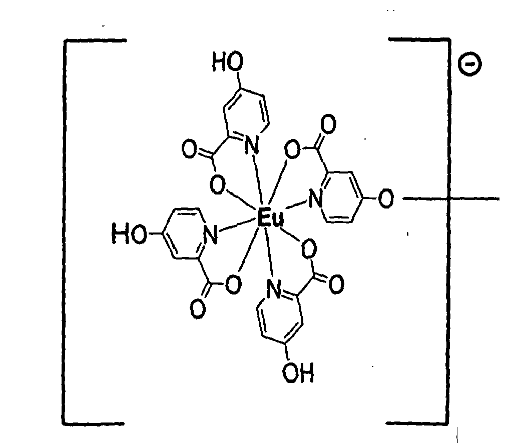 ist und/oder
**daß** der Fluorophor (B) ausgewählt ist aus Tetra(4-hydroxypyridin-2-carboxylato)europium(III), Tris(pyridin-2-carboxylato)(4-hydroxypyridin-2-carboxylato)europium(III), Bis(pyridin-2-carboxylato)-bis(4-hydroxypyridin-2-carboxylato)europium(III), (Pyridin-2-carboxylato)-tris(4-hydroxypyridin-2-carboxylato)europium(III) und/ oder dessen Derivaten und/oder
**daß** der Fluorophor (B) eine Verbindung der allgemeinen Formel
[Eu_{x'}(Pic')_{y'}(Pic'-Y')_{z'}]^{(4-3x')-}
ist, wobei in der vorbezeichneten Formel
• Eu Europium(III) ist,
• Pic' Picolinat ist,
• Y' eine funktionelle Gruppe ist, insbesondere ausgewählt aus der Gruppe von Amino-, Carboxylat-, Isocyanat-, Thioisocyanat-, Epoxy-, Thiol- und Hydroxygruppen, vorzugsweise einer Hydroxygruppe,
• x' eine ganze Zahl von 1 bis 4, insbesondere 1 oder 2, vorzugsweise 1, ist und
• y' und z' jeweils eine ganze Zahl von 0 bis 4 mit y' + z' = 4 sind.

6. Verwendung nach einem der vorangehenden Ansprüche, **dadurch gekennzeichnet,**
**daß** der zweiwertige und/oder zweibindige organische Rest ein Linker und/oder Spacer ist und/oder
**daß** der organische Rest ausgewählt wird derart, daß zwischen dem Fluorophor (A) und dem Fluorophor (B) ein Fluoreszenzenergietransfer eintreten kann, und/oder
**daß** der organische Rest an den jeweiligen Substituenten und/oder an die jeweilige funktionelle Gruppe des Liganden, insbesondere wie in den vorangehenden Ansprüchen definiert, gekoppelt und/oder gebunden ist und/oder
**daß** der organische Rest vorzugsweise kovalent mit dem Fluorophor (A) und/oder dem Fluorophor (B) verbunden ist, insbesondere wobei der organische Rest vorzugsweise kovalent an die funktionellen Gruppen des oder der Liganden des Fluorophors (A) und/oder (B) angebunden und/oder angekoppelt ist und/oder insbesondere wobei der organische Rest zur vorzugsweise kovalenten Bindung und/oder Kopplung an die funktionellen Gruppen des oder der Liganden fähige funktionelle Gruppen aufweist.

7. Verwendung einem der vorangehenden Ansprüche, **dadurch gekennzeichnet,**
**daß** der organische Rest eine Nukleotidsequenz, insbesondere eine DNA- oder RNA-Sequenz, vorzugsweise ein Oligonukleotid, ist oder diese umfaßt,
insbesondere wobei die Nukleotidsequenz, insbesondere an deren 5'-Ende und 3'-Ende, komplementäre Nukleotidsequenzabschnitte aufweist, insbesondere so daß bei nicht vorhandener Wechselwirkung mit einem Zielmolekül und/oder Zielstruktur eine Hybridisierung der komplementären Nukleotidsequenzabschnitte resultiert.

8. Verwendung nach einem der vorangehenden Ansprüche, **dadurch gekennzeichnet,**
**daß** der Fluorophor (A) und der Fluorophor (B) durch insbesondere kovalente Bindung an eine Nukleotidsequenz mit insbesondere an deren 5'-Ende und 3'-Ende komplementären Nukleotidsequenzabschnitten ein Molecular Beacon (MB) ausbilden und/oder
**daß** der Fluorophor (A) und der Fluorophor (B) mit einem organischen Rest ein Molekül der allgemeinen Formel gemäß Fig. 3 ausbilden, wobei in dieser Formel n eine ganze Zahl im Bereich von 1 bis 20, insbesondere 2 bis 15, vorzugsweise 2 bis 12, darstellt.

9. Fluoreszenzresonanzenergietransfer-Komplex (FRET-Komplex), aufweisend mindestens einen ersten Fluorophor (A) als Donorfluorophor und mindestens einen zweiten Fluorophor (B) als Akzeptorfluorophor,
wobei der erste Fluorophor (A) und der zweite Fluorophor (B), unabhängig voneinander, jeweils auf Basis eines Organometallkomplexes von Elementen der Seltenen Erden ausgebildet sind, wobei die Fluorophore (A) und (B) voneinander verschiedene Elemente der Seltenen Erden, ausgewählt aus der Gruppe von Europium und Terbium, aufweisen,
wobei die Elemente der Seltenen Erden in den Fluorophoren (A) und (B) jeweils mit mindestens einem Liganden in Form eines Komplex- und/oder Chelatbildners komplexiert sind, wobei der Ligand Picolinsäure, Picolinate und/oder deren Derivate ist und wobei der Ligand Substituenten und/oder funktionelle Gruppen zur vorzugsweise kovalenten Bindung und/oder Kopplung von vorzugsweise organischen Molekülen aufweist, wobei die Substituenten und/oder funktionellen Gruppen ausgewählt sind aus der Gruppe von Amino-, Carboxylat-, Isocyanat-, Thioisocyanat-, Epoxy-, Thiol- und Hydroxygruppen, und
wobei der Fluorophor (A) und der Fluorophor (B) über einen zweiwertigen und/oder zweibindigen organischen Rest miteinander gekoppelt und/oder verbunden sind.

10. Verwendung eines FRET-Komplexes nach Anspruch 9 zur Detektion eines Ereignisses in einer Probe.

11. Verwendung eines FRET-Komplexes nach Anspruch 9 zur Interaktion mit einem Zielmolekül und/oder einer Zielstruktur und/oder zur Detektion und/oder Identifikation und/oder zum Nachweis eines Zielmoleküls und/oder einer Zielstruktur, insbesondere durch Wechselwirkung des FRET-Komplexes und/oder des FRET-Systems einerseits mit dem Zielmolekül und/oder der Zielstruktur andererseits.

12. Verwendung eines FRET-Komplexes nach Anspruch 9 als Farbstoff, insbesondere Fluoreszenzfarbstoff, insbesondere für Markierungszwecke.

13. Verwendung eines FRET-Komplexes nach Anspruch 9 in oder als Biosensoren oder aber in oder als Sonden, insbesondere FRET-Sonden.

## Claims

1. Use of at least two fluorophores distinct from one another to create a fluorescence resonance energy transfer pair (FRET pair), wherein at least a first fluorophore (A) is used as the donor fluorophore and at least a second fluorophore (B) as the acceptor fluorophore within the FRET pair,
wherein the first fluorophore (A) and the second fluorophore (B), independently of one another, are each based on an organometallic complex of rare earth elements, wherein the fluorophores (A) and (B) exhibit rare earth elements which are distinct from one another, selected from the group of europium and terbium,
wherein the rare earth elements in the fluorophores (A) and (B) are each coordinated with at least one ligand to form a complexing and/or chelating agent, wherein the ligand is picolinic acid, a picolinate and/or derivatives thereof and wherein the ligand exhibits substituents and/or functional groups for the preferably covalent bonding and/or coupling of preferably organic molecules, wherein the substituents and/or functional groups are selected from the group of amino, carboxylate, isocyanate, thioisocyanate, epoxy, thiol and hydroxyl groups and
wherein the fluorophore (A) and the fluorophore (B) are coupled and/or connected to one another by a divalent and/or double-bonded organic residue.

2. The use according to Claim 1, **characterized in that**
the fluorophore (A) exhibits terbium in the form of terbium(III) as a rare earth metal and/or
the fluorophore (B) exhibits europium in the form of europium(III) as a rare earth metal.

3. The use according to Claim 1 or 2, **characterized in that**
the rare earth elements in the fluorophores (A) and (B) are coordinated with a plurality of ligands, preferably four ligands, and/or the rare earth elements in the fluorophores (A) and (B), independently of one another, are bonded ionically, coordinatively and/or covalently, particularly covalently, to the at least one ligand, particularly to a plurality of ligands, preferably to four ligands, and/or
the ligand is multidentate, particularly bidentate, in form and/or
the ligand is particularly a complexing agent and/or a chelating agent, picolinic acid, picolinate and/or derivatives thereof, particularly substituted derivatives, preferably hydroxyl derivatives, and/or
the ligand is hydroxypicolinic acid and/or hydroxypicolinate.

4. The use according to one of the preceding claims, **characterized in that**
the fluorophore (A) is an organometallic complex in accordance with the formula 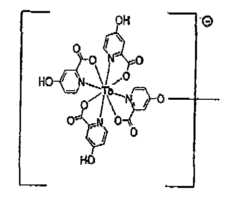 and/or
the fluorophore (A) is selected from tetra(4-hydroxypyridine-2-carboxylato)terbium(III), tris(pyridine-2-carboxylato)(4-hydroxypyridine-2-carboxylato)-terbium(III), bis(pyridine-2-carboxylato)-bis(4-hydroxypyridine-2-carboxylato)terbium(III), (pyridine-2-carboxylato)-tris(4-hydroxypyridine-2-carboxylato)-terbium(III) and/or derivatives thereof and/or that the fluorophore (A) is a compound with the general formula
[Tbₓ(Pic)_{y}(Pic-Y)_{z}]^{(4-3x)-},
wherein in the aforementioned formula
• Tb is terbium(III)
• Pic is picolinate
• Y is a functional group, particularly selected from the group of amino, carboxylate, isocyanate, thioisocyanate, epoxy, thiol and hydroxyl groups, preferably a hydroxyl group,
• x is a whole number between 1 and 4, particularly 1 or 2, preferably 1, and
• y and z are each a whole number between 0 and 4, where y + z = 4,

5. The use according to one of the preceding claims, **characterized in that**
the fluorophore (B) is an organometallic complex in accordance with the formula 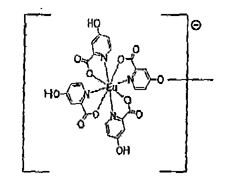 and/or
the fluorophore (B) is selected from tetra(4-hydroxypyridine-2-carboxylato)europium(III), tris(pyridine-2-carboxylato)(4-hydroxypyridine-2-carboxylato)europium(III), bis(pyridine-2-carboxylato)-bis(4-hyroxypyridine-2-carboxylato)europium(III), (pyridine-2-carboxylato)-tris(4-hydroxypyridine-2-carboxylato)europium(III) and/or derivatives thereof and/or
the fluorophore (B) is a compound with the general formula
[Euₓ(Pic')_{y'}(Pic'-Y')_{z'}]^{(4-3x')-}
wherein in the aforementioned formula
• Eu is europium(III),
• Pic' is picolinate,
• Y' is a functional group, particularly chosen from the group of amino, carboxylate, isocyanate, thioisocyanate, epoxy, thiol and hydroxyl groups preferably a hydroxyl group,
• x' is a whole number between 1 and 4, particularly 1 or 2, preferably 1, and
• y' and z' are each a whole number between 0 and 4 where y' +z' =4.

6. The use according to one of the preceding claims, **characterized in that**
the divalent and/or double-bonded organic residue is a linker and/or a spacer and/or
the organic residue is selected such that a fluorescence energy transfer can take place between the fluorophore (A) and the fluorophore (B) and/or
the organic residue is coupled and/or bonded to the respective substituents and/or to the respective functional group of the ligand, particularly as defined in the preceding claims, and/or
the organic residue is preferably covalently connected to the fluorophore (A) and/or the fluorophore (B), particularly wherein the organic residue is preferably covalently bonded and/or coupled to the functional groups of the ligand or ligands of the fluorophore (A) and/or (B) and/or particularly wherein the organic residue exhibits functional groups capable of preferably covalent bonding and/or coupling to the functional groups of the ligand or ligands.

7. The use according to one of the preceding claims, **characterized in that**
the organic residue is a nucleotide sequence, particularly a DNA or RNA sequence, preferably an oligonucleotide, or includes said sequence,
particularly wherein the nucleotide sequence, particularly at the 5' end and 3' end thereof, exhibits complementary nucleotide sequence sections, particularly so that hybridization of the complementary nucleotide sequence sections results where there is no interaction present with a target molecule and/or a target structure.

8. The use according to one of the preceding claims, **characterized in that**
the fluorophore (A) and the fluorophore (B) form a molecular beacon (MB) through particularly covalent bonding to a nucleotide sequence with complementary nucleotide sequence sections particularly at their 5' end and 3' end and/or
the fluorophore (A) and the fluorophore (B) form a molecule with the general formula according to Fig. 3 along with an organic residue, wherein in this formula n is a whole number in the range between 1 and 20, particularly 2 and 15, preferably 2 and 12.

9. A fluorescence resonance energy transfer complex (FRET complex) exhibiting at least a first fluorophore (A) as the donor fluorophore and at least a second fluorophore (B) as the acceptor fluorophore,
wherein the first fluorophore (A) and the second fluorophore (B), independently of one another, are each formed based on an organometallic complex of rare earth elements, wherein the fluorophores (A) and (B) exhibit rare earth elements which are distinct from one another, selected from the group of europium and terbium,
wherein the rare earth elements in the fluorophores (A) and (B) are each coordinated with at least one ligand in the form of a complexing and/or chelating agent, wherein the ligand is picolinic acid, picolinate and/or derivatives thereof and wherein the ligand exhibits substituents and/or functional groups for the preferably covalent bonding and/or coupling of preferably organic molecules, wherein the substituents and/or functional groups are selected from the group of amino, carboxylate, isocyanate, thioisocyanate, epoxy, thiol and hydroxyl groups and
wherein the fluorophore (A) and the fluorophore (B) are coupled and/or connected to one another by a divalent and/or double-bonded organic residue.

10. Use of a FRET complex according to Claim 9 for the detection of an event in a sample.

11. The use of a FRET complex according to Claim 9 for interaction with a target molecule and/or a target structure and/or for detection and/or identification and/or for detection of a target molecule and/or a target structure, particularly through interaction of the FRET complex and/or the FRET system firstly with the target molecule and/or secondly with the target structure.

12. The use of a FRET complex according to Claim 9 as a dyestuff, particularly a fluorescence dyestuff, particularly for marking purposes.

13. The use of a FRET complex according to Claim 9 in or as biosensors or, however, in or as probes, particularly FRET probes.

## Revendications

1. Utilisation d'au moins deux fluorophores différents l'un de l'autre pour la création d'une paire de transfert d'énergie de résonance par fluorescence (paire de FRET), à l'intérieur de la paire de FRET, au moins un premier fluorophore (A) faisant office de fluorophore donneur et au moins un deuxième fluorophore (B) faisant office de fluorophore accepteur,
indépendamment l'un de l'autre, le premier fluorophore (A) et le deuxième fluorophore (B) étant conçus chacune sur la base d'un complexe organométallique d'éléments de terres rares, les fluorophores (A) et (B) présentant des éléments différents l'un de l'autre des terres rares, choisis dans le groupe de l'europium et du terbium,
les éléments des terres rares dans les fluorophores (A) et (B) étant chacun complexé avec au moins un ligand sous la forme d'un agent complexant et/ou chélateur, le ligand étant de l'acide picolinique, des picolinates et/ou leurs dérivés et le ligand comportant des substituants et/ou des groupes fonctionnels pour la liaison et/ou le couplage de préférence covalents de molécules de préférence organiques, les substituants et/ou les groupes fonctionnels étant choisis dans le groupe comprenant les groupes amino, les groupes carboxylate, les groupes isocyanates, les groupes thioisocyanates, les groupes époxy, les groupes thioles et les groupes hydroxy et
le fluorophore (A) et le fluorophore (B) étant couplés et/ou reliés par l'intermédiaire d'un radical organique bivalent ou divalent.

2. Utilisation selon la revendication 1, **caractérisée en ce que**
le fluorophore (A) comporte en tant que métal des terres rares du terbium sous le forme de terbium(III) et/ou
le fluorophore (B) comporte en tant que métal des terres rares de l'europium sous la forme d'europium(III).

3. Utilisation selon la revendication 1 ou 2, **caractérisée en ce que**
les éléments des terres rares dans les fluorophores (A) et (B) sont complexés avec plusieurs ligands, de préférence quatre ligands et/ou **en ce qu'**indépendamment les uns des autres, les éléments des terres rares dans les fluorophores (A) et (B) sont liés par liaison ionique, coordonnée et/ou covalente, notamment covalente à l'au moins un ligand, notamment à plusieurs ligands, de préférence à quatre ligands et/ou
**en ce que** le ligand est conçu sous forme multidentate, notamment bidentate et/ou
**en ce que** le ligand est notamment un agent complexant et/ou chélateur, de l'acide picolinique, des picolinates et/ou leur dérivés, notamment des dérivés substitués, de préférence des dérivés hydroxy et/ou
**en ce que** le ligand est de l'acide hydroxypicolinique et/ou un hydroxypicolinate.

4. Utilisation selon l'une quelconque des revendications précédentes, **caractérisée en ce que**
le fluorophore (A) est un complexe organométallique selon la formule 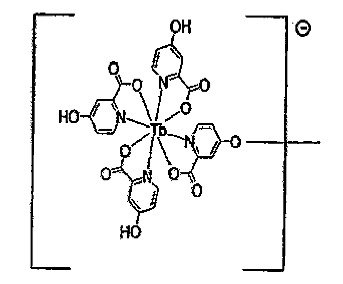 et/ou
**en ce que** le fluorophore (A) est choisi parmi le tétra(4-hydroxypiridine-2-carboxylato)terbium(III), le tris(pyridine-2-caxboxylato)(4(hydroxypyridine-2-carboxylato)terbium(III), le bis(pyridine-2-carboxylato)bis(4(hydroxypyridine-2-carboxylato)terbium(III), le (pyridine-2-carboxylato)-tris(4(hydioxypyridine-2-carboxylato)terbium(III) et/ou ses dérivés et/ou **en ce que** le fluorophore (A) est un composé de la formule générale
[Tbₓ(Pic)_{y}(Pic-Y)_{z}]^{(4-3x)-},
dans la formule précédemment citée,
• Tb étant du terbium(III)
• Pic étant un picolinate
• Y étant un groupe fonctionnel, notamment choisi dans le groupe des groupes amino, des groupes carbaxylates, des groupes isocyanates, des groupes thioisocyanates, des groupes époxy, des groupes thioles et des groupes hydroxy, de préférence dans un groupe hydroxy
• x étant un nombre entier de 1 à 4, notamment 1 ou 2, de préférence 1 et
• y et z étant chacun un nombre entier de 0 à 4, avec y + z= 4.

5. Utilisation selon l'une quelconque des revendications précédentes, **caractérisée en ce que**
le fluorophore (B) est un complexe organométallique selon la formule 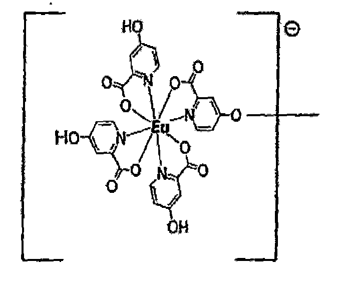 et/ou
**en ce que** le fluorophore (B) est choisi parmi le tétra(4-hydroxypiridine-2-carboxylato)europium(III), le tris(pyridine-2-carboxylato)(4(hydroxypyridine-2-carboxylato)europium(III), le bis(pyridine-2-carboxylato)bis(4(hydroxypyridine-2-carboxylato)europium(ITI), le (pyridine-2-carboxylato)-tris(4(hydroxypyridine-2-carboxylato)europium(III) et/ou ses dérivés et/ou
**en ce que** le fluorophore (B) est un composé de la formule générale
[Eu_{x'}(Pic')_{y'}(Pic'-Y')_{2'}]^{(4-3x')-}
dans la formule précédemment citée,
• Eu étant de l'europium(III)
• Pic étant un picolinate
• Y étant un groupe fonctionnel, notamment choisi dans le groupe des groupes amino, des groupes carboxylates, des groupes isocyanates, des groupes thioisocyanates, des groupes époxy, des groupes thioles et des groupes hydroxy, de préférence dans un groupe hydroxy
• x' étant un nombre entier de 1 à 4, notamment 1 ou 2, de préférence 1 et
• y' et z' étant chacun un nombre entier de 0 à 4, avec y' +z' = 4.

6. Utilisation selon l'une quelconque des revendications précédentes, **caractérisée en ce que**
le radical organique bivalent et/ou divalent est un lieur et/ou un espaceur et/ou
**en ce que** le radical organique est choisi de sorte qu'entre le fluorophore (A) et le fluorophore (B), il puisse se produire un transfert d'énergie de résonance par fluorescence et/ou
**en ce que** le radical organique est couplé et/ou lié aux substituants respectifs et/ou au groupe fonctionnel respectif du ligand, notamment tel que défini dans les revendications précédentes et/ou
**en ce que** le radical organique est reliée de manière de préférence covalente au fluorophore (A) et/ou au fluorophore (B), notamment le radical organique étant lié et/ou couplé de manière de préférence covalente aux groupes fonctionnels du ou des ligands du fluorophore (A) et/ou (B) et/ou le radical organique comportant des groupes fonctionnels aptes notamment pour la liaison et/ou le couplage de préférence covalents aux groupes fonctionnels du ou des ligands.

7. Utilisation selon l'une quelconque des revendications précédentes, **caractérisée en ce que** le radical organique est une séquence de nucléotides, notamment une séquence d'ADN ou d'ARN, **en ce qu'**il est ou comprend de préférence un oligonucléotide,
notamment la séquence de nucléotides comprenant à sa fin 5' et à sa fin 3' des portions complémentaire de séquence de nucléotides, notamment de sorte qu'en cas d'absence d'interaction avec une molécule cible et/ou une structure cible, il résulte une hybridation des portions complémentaires de séquence de nucléotides.

8. Utilisation selon l'une quelconque des revendications précédentes, **caractérisée en ce que**
par liaison notamment covalente à la séquence de nucléotides avec notamment à leur fin 5' et à leur fin 3' des portions complémentaires de séquence de nucléotides, le fluorophore (A) et le fluorophore (B) forment un Molecular Beacon (MB) et/ou
**en ce qu'**avec le radical organique, le fluorophore (A) et le fluorophore (B) forment une molécule de la formule générale selon la figure 3, dans ladite formule, n représentant un nombre entier de l'ordre de 1 à 20, notamment de 2 à 15, de préférence de 2 à 12.

9. Complexe de transfert d'énergie de résonance par fluorescence (complexe de FRET), comportant au moins un premier fluorophore (A), en tant que fluorophore donneur et au moins un deuxième fluorophore (B), en tant que fluorophore accepteur,
indépendamment l'un de l'autre, le premier fluorophore (A) et le deuxième fluorophore (B) étant conçus chacune sur la base d'un complexe organométallique d'éléments de terres rares, les fluorophores (A) et (B) présentant des éléments différents l'un de l'autre des terres rares, choisis dans le groupe de l'europium et du terbium,
les éléments des terres rares dans les fluorophores (A) et (B) étant chacun complexé avec au moins un ligand sous la forme d'un agent complexant et/ou chélateur, le ligand étant de l'acide picolinique, des picolinates et/ou leurs dérivés et le ligand comportant des substituants et/ou des groupes fonctionnels pour la liaison et/ou le couplage de préférence covalents de molécules de préférence organiques, les substituants et/ou les groupes fonctionnels étant choisis dans le groupe comprenant les groupes amino, les groupes carboxylates, les groupes isocyanates, les groupes thioisocyanates, les groupes époxy, les groupes thioles et les groupes hydroxy et
le fluorophore (A) et le fluorophore (B) étant couplés et/ou reliés par l'intermédiaire d'un radical organique bivalent ou divalent.

10. Utilisation d'un complexe de FRET selon la revendication 9 pour la détection d'un événement dans une éprouvette.

11. Utilisation d'un complexe de FRET selon la revendication 9 pour l'interaction avec une molécule cible et/ou une structure cible et/ou pour la détection et/ou l'identification et/ou pour la mise en évidence d'une molécule cible et/ou d'une structure cible, notamment par interaction du complexe de FRET et/ou du système de FRET, d'une part avec la molécule cible et/ou d'autre part avec la structure cible.

12. Utilisation d'un complexe de FRET selon la revendication 9 en tant que colorant, notamment en tant que colorant fluorescent, notamment à des fins de marquage.

13. Utilisation d'un complexe de FRET selon la revendication 9 dans des biocapteurs ou en tant que tels ou encore dans des sondes ou en tant que telles, notamment dans des sondes de FRET.
